(19) Europäisches Patentamt
European Patent Office
Office européen des brevets

(11) EP 3 135 763 A1

(12) EUROPEAN PATENT APPLICATION
published in accordance with Art. 153(4) EPC

(43) Date of publication:
**01.03.2017 Bulletin 2017/09**

(21) Application number: **15782362.6**

(22) Date of filing: **23.04.2015**

(51) Int Cl.:
***C12N 15/09*** (2006.01) ***A61K 31/7105*** (2006.01)
***A61K 48/00*** (2006.01) ***A61P 25/00*** (2006.01)
***A61P 25/02*** (2006.01) ***A61P 43/00*** (2006.01)

(86) International application number:
**PCT/JP2015/002208**

(87) International publication number:
**WO 2015/162930 (29.10.2015 Gazette 2015/43)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **24.04.2014 JP 2014090634**

(71) Applicant: **Accurna, Inc.**
**Tokyo 113-0033 (JP)**

(72) Inventors:
- **ITAKA, Keiji**
  **Tokyo 113-0033 (JP)**
- **KATAOKA, Kazunori**
  **Tokyo 113-0033 (JP)**
- **IKEGAMI, Masaru**
  **Tokyo 113-0033 (JP)**
- **UCHIDA, Satoshi**
  **Tokyo 113-0033 (JP)**
- **UCHIDA, Hirokuni**
  **Tokyo 113-0033 (JP)**
- **NAGATA, Kazuya**
  **Tokyo 113-0033 (JP)**

(74) Representative: **Hoffmann Eitle**
**Patent- und Rechtsanwälte PartmbB**
**Arabellastraße 30**
**81925 München (DE)**

# (54) METHOD FOR IMPROVING PROTEIN EXPRESSION, AND COMPOSITION FOR PROTEIN EXPRESSION

(57) The present invention provides a method for improving protein expression and a composition for protein expression. The composition is a composition for use in expressing a target protein, the composition comprising an mRNA encoding the target protein, wherein 80% or more of the mRNA molecules contained in the composition have a sequence consisting substantially of poly(A) having a length of 230 to 250 nucleotides on the 3'-end side of the protein-coding region thereof.



Printed by Jouve, 75001 PARIS (FR)

## Description

Cross-reference to related application

This application claims the benefit of priority of Japanese Patent Application No. 2014-090634, filed April 24, 2014, which is hereby incorporated by reference herein in its entirety.

Technical Field

The present invention relates to a method for improving protein expression and a composition for protein expression.

Background Art

Proteins have extremely great potential as physiologically active substances. For example, in the treatment of a disease caused by a decrease in a specific protein, protein replacement treatment exhibits advantageous effects. Accordingly, techniques for intracellularly or extracellularly producing large amounts of proteins have been developed until now.

A protein is generated by transcription of a DNA encoding the protein into an mRNA and translation of the mRNA into the protein. In the transcription of a DNA into an mRNA, a transcription regulator is involved in and controls the production amount of the mRNA by transcriptional regulation. In contrast, the translation of an mRNA into a protein is assumed to be controlled by, for example, association of a translation initiation factor with the mRNA. It is also known that mRNAs are unstable in cells and that the stability is changed by poly(A) added to the 3'UTR of an mRNA.

Poly(A) is normally added to the 3'UTR of an mRNA by a poly(A) addition signal. Specifically, it is assumed that transcription of a DNA into an mRNA by RNA polymerase II allows a complex containing a cleavage and polyadenylation specificity factor (CPSF) to recognize the poly(A) addition signal region of 3'UTR of the mRNA to cleave the mRNA at a position 10 to 30 nucleotides downstream from the signal and thereby to start polyadenylation from the cleavage site, and that the synthesis of poly(A) is terminated when poly(A) reaches about 100 to 300 nucleotides (Non Patent Literature 1). However, the synthesis of poly(A) is not strictly controlled.

The length of poly(A) is assumed to be involved in the quantity of translation to a protein from an mRNA. For example, Non Patent Literature 2 discloses that the translation efficiency of an mRNA was enhanced when the length of poly(A) added to the 3'UTR of the mRNA was 120 nucleotides. However, synthesis of poly(A) having a controlled length is difficult, and the study has not progressed any more. At present, the actual situation is that poly(A) is enzymatically added to an mRNA based on the poly(A) addition signal region. The expression level is controlled by selecting a promoter, and various expression vectors for enhanced expression of proteins due to improvement in promoter sequence have come to the market.

Citation List

Non Patent Literature

Non Patent Literature 1: E. Wahle, Journal of Biological Chemistry, 270: 2800-2808, 1995
Non Patent Literature 2: Holtkamp et al., Blood, 108: 4009-4017, 2006

Summary of Invention

The present invention provides a method for improving protein expression and a composition for protein expression.

The present inventors have found that an mRNA having a poly(A) length in a certain range can significantly enhance its binding to eukaryotic translation initiation factor 4E (eIF4E). The present inventors have also found that an mRNA having a poly(A) length in a certain range exhibits notably high translation efficiency. The present invention is based on these findings.

The present invention provides the following aspects:

(1) A composition for use in expressing a target protein, the composition comprising:

an mRNA encoding the target protein, wherein
80% or more of the mRNA molecules contained in the composition have a sequence consisting substantially of poly(A) having a length of 230 to 250 nucleotides on the 3'-end side of the protein-coding region thereof;

(2) The composition according to above (1), wherein 90% or more of the mRNA molecules contained in the composition have a sequence consisting substantially of poly(A) having a length of 230 to 250 nucleotides on the 3'-end side of the protein-coding region thereof;
(3) The composition according to above (1), wherein 95% or more of the mRNA molecules contained in the composition have a sequence composed substantially of poly(A) having a length of 230 to 250 nucleotides on the 3'-end side of the protein-coding region thereof;
(4) The composition according to any one of above (1) to (3), wherein 20% or less of the mRNA molecules contained in the composition have poly(A) having a length of 270 or more nucleotides;
(5) A protein expression vector comprising:

a gene encoding a protein and operably linked to a promoter; and
a sequence consisting substantially of poly(A) having a length of 230 to 250 nucleotides downstream the protein-coding region of the gene encoding the protein;

(6) A method for improving an ability of an mRNA to bind to eIF4E, comprising:

adding a sequence consisting of poly(A) or consisting substantially of poly(A) to the downstream of the protein-coding region of a DNA to be transcribed into the mRNA such that the mRNA has a sequence consisting substantially of poly(A) having a length of 230 to 250 nucleotides on the 3'-end side of the protein-coding region thereof;

(7) A method for expressing a target protein in a cell in the body of a subject, comprising:

providing a composition comprising an mRNA encoding the target protein, where 80% or more of the mRNA molecules contained in the composition have a sequence consisting substantially of a poly(A) sequence having a length of 230 to 250 nucleotides on the 3'-end side of the protein-coding region of the mRNA; and
administering the composition to the subject;

(8) A pharmaceutical composition for use in treating a disease or a disorder in a subject suffering from the disease or a subject having the disorder, the pharmaceutical composition comprising an mRNA encoding a protein that can treat the disease or the disorder, wherein 80% or more of the mRNA molecules contained in the composition have a sequence composed substantially of a poly(A) sequence having a length of 230 to 250 nucleotides on the 3'-end side of the protein-coding region of the mRNA;
(9) The composition according to above (8), wherein the disease or the disorder is a disease or a disorder caused by a reduction or a lack of a protein, and the protein that can treat the disease or the disorder is the protein that is reduced or lacked in the subject;
(10) The composition according to above (8), wherein the disease or the disorder is spinal cord injury, and the protein that can treat the disease or the disorder is brain-derived neurotrophic factor (BDNF); and
(11) The composition according to above (8), wherein the disease or the disorder is peripheral nerve injury, and the protein that can treat the disease or the disorder is insulin-like growth factor (IGF-1).

Brief Description of Drawings

[Figure 1] Figure 1 is an electrophoresis photograph of purified mRNAs.
[Figure 2] Figure 2 includes graphs showing the effects of poly(A) length on the quantity of binding of an mRNA to the proteins shown in the graphs.
[Figure 3] Figure 3 is a graph showing a relationship between the poly(A) length and the translation efficiency in cultured cells.
[Figure 4] Figure 4 includes graphs showing relationships between the poly(A) length and the translation efficiency in cell-free extract systems, where Figure 4A shows the results in a human cell-free extract system, and Figure 4B shows the results in a rabbit reticulocyte lysate.
[Figure 5] Figure 5 is a graph showing a relationship between the poly(A) length and the translation efficiency *in vivo.*

[Figure 6] Figure 6 includes diagrams showing the influence of poly(A) length on the therapeutic effect in a sciatic nerve damage model.

Description of Embodiments

In the present invention, the term "mRNA" refers to messenger RNA. The mRNA is preferably derived from a eukaryote. Examples of the eukaryote include bacteria, such as *Escherichia coli;* fungi, such as yeast; insects, such as silkworm; and mammals, such as human. The eukaryote is more preferably *Escherichia coli,* yeast, silkworm, or human. In a living organism, an mRNA is generated by transcription from a DNA. After the completion of the transcription, poly(A) is added to the 3'-end of the mRNA. In the mRNA, 3' untranslated region (3'UTR) normally lies between the protein-coding region (CDS) and the poly(A).

As used herein, the term "protein-coding region" refers to a region, on a DNA, encoding a protein or a region, on an RNA, encoding a protein. The term "3'-end side of the protein-coding region" refers to, in an mRNA, a region outside the protein-coding region and lying on the 3'-end side and preferably refer to a region further outside the 3'UTR, and may be meant to the 3'-end of the 3'UTR.

As used herein, the term "upstream" refers to a region lying on the opposite side of the direction of reading genetic information when viewed from a protein-coding region of a gene. As used herein, the term "downstream" refers to a region lying in the direction of reading genetic information when viewed from a protein-coding region of a gene.

Herein, the term "poly(A)" refers to a DNA or an RNA made by polymerization of adenine. Herein, the term "sequence consisting substantially of poly(A)" represents that 90% or more, preferably 95% or more, more preferably 97% or more, and most preferably 100% of all nucleotides of the sequence consist of adenine (A). That is, a sequence consisting substantially of poly(A) may contain nucleotides other than adenine (A). In a sequence consisting substantially of poly(A), the nucleotides other than adenine (A) is concentrated in, for example, three or less, preferably two or less, and more preferably one region. In a sequence consisting substantially of poly(A), the nucleotides other than adenine (A) are, for example, restriction enzyme cleavage sites.

Herein, the term "promoter" refers to a transcriptional regulatory region for an mRNA lying on a DNA. In the present invention, *in vitro* transcriptional promoters and eukaryotic promoters can be used as the "promoter". Examples of the *in vitro* transcriptional promoter include SP6 promoter, T3 promoter, and T7 promoter, and these promoters can be used in the present invention. Examples of the eukaryotic promoter include promoters of bacteria such as *Escherichia coli* and promoters of mammals, and promoters appropriately selected depending on the host cell can be used in the present invention. Examples of the promoter for *Escherichia coli* include T7 promoter, tac promoter, and T7 lac promoter. Examples of the promoter for mammals include CMV promoter; $\beta$ actin promoters, such as CAG promoter; EF1$\alpha$ promoter; and SR$\alpha$ promoter. Herein, the term "terminator" refers to the termination signal of transcription.

A process of polyadenylation in general translation of an mRNA *in vivo* can be described as follows. It is assumed that transcription of a DNA into an mRNA by RNA polymerase II allows a complex containing a cleavage and polyadenylation specificity factor (CPSF) to recognize the poly(A) addition signal region of 3'UTR of the mRNA to cleave the mRNA downstream the signal and thereby to start polyadenylation from the cleavage site.

A poly(A) tail functions as a binding site for a poly(A)-binding protein and accelerates the transport of the mRNA to the outside of the nucleus. The poly(A) tail also has a role of protecting the mRNAs from degradation in cytoplasm. Consequently, the poly(A) tail contributes to an improvement in efficiency of translation into a protein.

eIF4E, also called Eukaryotic translation initiation factor 4E, has important roles in the translational control of an mRNA, involving in recognition of the 5' cap structure of the mRNA and playing a role in circularization of the mRNA through eIF4G and poly(A)-binding protein (PABP).

The present inventors have found that although the strength of binding of an mRNA having long poly(A) to PABP increases, the formation of a complex of the mRNA and eIF4E is significantly enhanced at a specific poly(A) length (specifically, 240 nucleotides). In addition, if the poly(A) length is longer than a certain value, the strength of binding to PABP is increased, but the strength of binding to eIF4E is decreased. Since it is assumed that eIF4E binds to (or forms a complex with) the poly(A) of an mRNA through PABP, the result that an mRNA having long poly(A), which strengthens the binding to PABP, weakens the binding to eIF4E is very surprising.

In addition, according to the present invention, when the poly(A) length of an mRNA is 210 or less nucleotides or 270 or more nucleotides, the translation efficiency is significantly decreased compared with a case of 240 nucleotides. In the present invention, therefore, the poly(A) length of an mRNA is preferably controlled to a certain length. The term "a certain length" herein is, for example, 220 to 260 nucleotides, preferably 225 to 255 nucleotides, particularly preferably 230 to 250 nucleotides, further preferably 230 to 245 nucleotides, and most preferably 235 to 245 nucleotides. In a composition of a certain embodiment, mRNAs having poly(A) of 270 or more nucleotides are reduced in their amounts or are removed.

The poly(A) length can be easily controlled by removing the poly(A) addition signal. Accordingly, in a certain embodiment of the present invention, the mRNA does not have a poly(A) addition signal. In another embodiment of the

present invention, the DNA that is transcribed into an mRNA does not have a poly(A) addition signal between the protein-coding region and the terminator. In a certain another embodiment, the DNA that is transcribed into an mRNA does not have a poly(A) addition signal between the protein-coding region and the terminator, but instead has a sequence consisting substantially of poly(A) having a certain length.

The present inventors have revealed that an mRNA including poly(A) having a certain length has improved ability to bind to eIF4E. Accordingly, an aspect of the present invention provides a method for improving the ability of an mRNA to bind to eIF4E, wherein the method comprises adding a sequence consisting of poly(A) or consisting substantially of poly(A) to the downstream side of the protein-coding region of a DNA to be transcribed into an mRNA such that the mRNA has a sequence consisting substantially of poly(A) having a certain length on the 3'-end side of the protein-coding region there. In a preferred embodiment, the certain length is of 230 to 250 nucleotides.

An mRNA is given by transcription of the region between the transcription start region and the terminator on a DNA by RNA polymerase II. Accordingly, a sequence consisting of poly(A) or consisting substantially of poly(A) can be inserted into between the protein-coding region and the terminator of a DNA.

The method for improving the ability of an mRNA to bind to eIF4E according to the present invention may further comprise removing of the poly(A) addition signal. The poly(A) addition signal is typically AATAAA (or AAUAAA), and those skilled in the art can readily remove the poly(A) addition signal. The poly(A) addition signal is contained in the region between the CDS and the terminator or in the 3'UTR and therefore can be removed also by removing a part or whole of these regions.

In a certain embodiment, the method for improving the ability of an mRNA to bind to eIF4E according to the present invention may further comprise reducing or removing mRNAs including poly(A) having a length of 270 or more nucleotides. In a certain embodiment of the present invention, the method may further comprise reducing or removing mRNAs including poly(A) having a length of 210 or less nucleotides. In a certain embodiment of the present invention, the method may further comprise reducing or removing mRNAs including poly(A) having a length of 210 or less nucleotides and a length of 270 or more nucleotides.

The present inventors have revealed that the translation efficiency is significantly improved when the mRNA has a poly(A) length of 240 nucleotides. The translation efficiency was notably higher than the efficiencies of an mRNA having a poly(A) length of 210 nucleotides and an mRNA having a poly(A) length of 270 nucleotides. Since it has been conventionally assumed that the stability increases and the translation efficiency is enhanced with the length of poly(A), it was unexpected that the translation efficiency of an mRNA increases within a specific and significantly narrow range of the poly(A) length and that the increase is notable.

Accordingly, an aspect of the present invention provides a composition for use in expressing a target protein, comprising an mRNA encoding a target protein, and 80% or more (molecular number rate, the same applies hereinafter), preferably 90% or more, more preferably 95% or more, further preferably 97% or more, further more preferably 99% or more, and most preferably 100% of the mRNA molecules contained in the composition have a sequence consisting substantially of poly(A) having a certain length (for example, 230 to 250 nucleotides) on the 3'-end side of the protein-coding region thereof. As described above, the poly(A) length of the mRNA is preferably controlled to a certain length. The molecular number rate (%) of an mRNA can be determined by a method well known to those skilled in the art. The molecular number rate can be determined, for example, from an electrophoresis pattern prepared by electrophoresis of the mRNA encoding the target protein. In addition, electrophoresis showing excellent quantitativeness by an RNA analysis microchip has been developed and can be used for calculation of the molecular number rate of an mRNA.

In a certain embodiment, the composition of the present invention contains mRNA molecules encoding the target protein contained in the composition and including poly(A) having a length of 270 or more nucleotides in an amount of 20% or less, preferably 15% or less, more preferably 10% or less, further preferably 5% or less, further more preferably 3% or less, and particularly preferably 1% or less, and most preferably, the composition does substantially not contain mRNA molecules encoding the target protein and including poly(A) having a length of 270 or more nucleotides. In a certain embodiment, the composition of the present invention contains mRNA molecules encoding the target protein and including poly(A) having a length of 210 or less nucleotides in an amount of 20% or less, preferably 15% or less, more preferably 10% or less, further preferably 5% or less, further more preferably 3% or less, and particularly preferably 1% or less, and most preferably, the composition does substantially not contain mRNA molecules encoding the target protein and including poly(A) having a length of 210 or less nucleotides. In a certain embodiment of the present invention, the composition contains the mRNA molecules encoding the target protein and including poly(A) having a length of 210 or less nucleotides and a length of 270 or more nucleotides in an amount of 20% or less, preferably 15% or less, more preferably 10% or less, further preferably 5% or less, further more preferably 3% or less, and particularly preferably 1% or less, and most preferably, the composition does substantially not contain mRNA molecules including poly(A) having a length of 210 or less nucleotides or 270 or more nucleotides.

In naturally occurring mRNAs or mRNAs occurring by addition of poly(A) by a poly(A) addition signal, the length of poly(A) distributes in a broad range. In contrast, in the composition of the present invention, 80% or more of the mRNA molecules encoding a target protein contained in the composition have a certain poly(A) length (for example, 230 to 250

nucleotides). If the poly(A) has a certain length (for example, 230 to 250 nucleotides), the translation efficiency is significantly increased, which is extremely advantageous from the viewpoint of translation efficiency. In a preferred embodiment of the present invention, 90% or more, further preferably 95% or more, further more preferably 97% or more, particularly preferably 99% or more, and most preferably 100% of the mRNA molecules encoding a target protein contained in the composition have a sequence consisting substantially of poly(A) having a certain length (for example, 230 to 250 nucleotides) on the 3'-end side of the protein-coding region thereof. In a certain embodiment of the present invention, the mRNA does not have a poly(A) addition signal.

The composition of the present invention may contain one kind of mRNA or may be a mixture of a plurality of kinds of mRNAs. The composition of the present invention may be a mixture of mRNAs having a variety of lengths of poly(A). The composition of the present invention may contain a carrier, such as a buffer solution, or an excipient, in addition to the mRNA. The composition of the present invention may contain a carrier for mRNA delivery.

The composition of the present invention can be used for expressing a protein *in vitro* or in *in vivo* cells. The composition of the present invention can also be used for expressing a protein in an *in vitro* translation system, such as a cell-free extract system.

An aspect of the present invention provides a protein expression vector including a gene encoding a protein and operably linked to a promoter and including a sequence consisting substantially of poly(A) having a certain length (for example, 230 to 250 nucleotides) downstream the protein-coding region of the gene encoding the protein. The protein expression vector of the present invention is used in production of an mRNA to which a sequence consisting substantially of poly(A) having a certain length (for example, 230 to 250 nucleotides) in the 3'UTR region is added. In a certain embodiment of the present invention, no poly(A) addition signal lies between the CDS and the terminator.

An aspect of the present invention provides a method for expressing a target protein in a cell in the body of a subject, comprising providing a composition containing an mRNA encoding the target protein, where 80% or more, preferably 90% or more, more preferably 95% or more, further preferably 97% or more, further more preferably 99% or more, and most preferably 100% of the mRNA molecules contained in the composition have a sequence consisting substantially of a poly(A) sequence having a certain length (for example, 230 to 250 nucleotides) on the 3'-end side of the protein-coding region of the mRNA; and administering the composition to the subject.

In a certain embodiment, the method for expressing a target protein in a cell in the body of a subject according to the present invention may further comprise reducing or removing mRNA molecules including poly(A) having a length of 270 or more nucleotides. In a certain embodiment of the present invention, the method may further comprise reducing or removing mRNA molecules including poly(A) having a length of 210 or less nucleotides. In a certain embodiment of the present invention, the method may further comprise reducing or removing mRNA molecules including poly(A) having a length of 210 or less nucleotides and 270 or more nucleotides.

Another aspect of the present invention provides a method for treating a disease or a disorder in a subject suffering from the disease or a subject having the disorder, comprising administering to the subject a composition containing an mRNA encoding a protein that can treat the disease or the disorder, where 80% or more, preferably 90% or more, more preferably 95% or more, further preferably 97% or more, further more preferably 99% or more, and most preferably 100% of the mRNA molecules contained in the composition have a sequence consisting substantially of a poly(A) sequence having a certain length (for example, 230 to 250 nucleotides) on the 3'-end side of the protein-coding region of the mRNA.

In a specific embodiment of the method for treating a disease or a disorder according to the present invention, the disease or the disorder is peripheral nerve injury, and the protein that can treat the disease or the disorder is insulin-like growth factor (IGF-1). In a specific embodiment of the present invention, the peripheral nerve injury can be a sciatic nerve damage. IGF-1 exhibits a muscle hypertrophy effect (or muscle atrophy preventing effect) by, for example, intramuscular administration of an mRNA encoding IGF-1, has a nerve regeneration enhancing action, accelerates regeneration of damaged nerve, and can recover the motor function of the subject.

In another specific embodiment of the method for treating a disease or a disorder according to the present invention, the disease or the disorder is a spinal cord injury, and the protein that can treat the disease or the disorder is brain-derived neurotrophic factor (BDNF). BDNF enhances the recovery of nervous function after the spinal cord injury by, for example, intrathecal administration of an mRNA encoding BDNF.

In another specific embodiment of the method for treating a disease or a disorder of the present invention, the disease or the disorder is a disease or a disorder caused by a reduction or a lack of a protein, and the protein that can treat the disease or the disorder is the protein that is reduced or lacked in the disease or the disorder. That is, this specific embodiment is protein replacement treatment. In another specific embodiment of the method for treating a disease or a disorder of the present invention, the protein that can treat the disease or the disorder is a secretory factor or an accelerator accelerating the production of a secretory factor, and the disease or the disorder is a disease or a disorder caused by a reduction or a lack of the secretory factor.

Another aspect of the present invention provides a pharmaceutical composition for use in the method for treating a disease or a disorder according to the present invention. That is, the present invention provides a pharmaceutical

composition for use in treating a disease or a disorder in a subject suffering from the disease or a subject having the disorder, comprising an mRNA encoding a protein that can treat the disease or the disorder, where 80% or more, preferably 90% or more, more preferably 95% or more, further preferably 97% or more, further more preferably 99% or more, and most preferably 100% of the mRNA molecules contained in the composition have a sequence consisting substantially of a poly(A) sequence having a certain length (for example, 230 to 250 nucleotides) on the 3'-end side of the protein-coding region of the mRNA. In a specific embodiment of the pharmaceutical composition of the present invention, the disease or the disorder is peripheral nerve injury, and the protein that can treat the disease or the disorder is insulin-like growth factor (IGF-1). In a specific embodiment of the present invention, the peripheral nerve injury can be a sciatic nerve damage. In another specific embodiment of the pharmaceutical composition of the present invention, the disease or the disorder is spinal cord injury, and the protein that can treat the disease or the disorder is brain-derived neurotrophic factor (BDNF).

Another embodiment of the pharmaceutical composition of the present invention provides a pharmaceutical composition for treating and/or preventing a disease or a disorder caused by a reduction or a lack of a protein, comprising an mRNA encoding the protein, where 80% or more, preferably 90% or more, more preferably 95% or more, further preferably 97% or more, further more preferably 99% or more, and most preferably 100% of the mRNA molecules contained in the composition have a sequence consisting substantially of a poly(A) sequence having a certain length (for example, 230 to 250 nucleotides) on the 3'-end side of the protein-coding region of the mRNA. In a specific embodiment, in the pharmaceutical composition of the present invention, the protein is a secretory factor.

Another aspect of the present invention relates to a use of an mRNA in production of a pharmaceutical composition for use in treating a disease or a disorder in a subject suffering from the disease or a subject having the disorder, wherein the mRNA encodes a protein that can treat the disease or the disorder and have a sequence consisting substantially of a poly(A) sequence having a certain length (for example, 230 to 250 nucleotides) on the 3'-end side of the protein-coding region of the mRNA. A specific embodiment of the present invention provides a use of a composition in production of a pharmaceutical composition for use in treating a disease or a disorder in a subject suffering from the disease or a subject having the disorder, wherein the composition comprises an mRNA encoding a protein that can treat the disease or the disorder, and wherein 80% or more, preferably 90% or more, more preferably 95% or more, further preferably 97% or more, further more preferably 99% or more, and most preferably 100% of the mRNA molecules contained in the composition have a sequence consisting substantially of a poly(A) sequence having a certain length (for example, 230 to 250 nucleotides) on the 3'-end side of the protein-coding region of the mRNA. In a specific embodiment of the use of the present invention, the disease or the disorder is peripheral nerve injury, and the protein that can treat the disease or the disorder is insulin-like growth factor (IGF-1). In a specific embodiment of the present invention, the peripheral nerve injury can be a sciatic nerve damage. In another specific embodiment of the use of the present invention, the disease or the disorder is spinal cord injury, and the protein that can treat the disease or the disorder is brain-derived neurotrophic factor (BDNF). In another embodiment of the use of the present invention, the disease or the disorder is a disease or a disorder caused by a reduction or a lack of a protein, and the protein that can treat the disease or the disorder is the protein that is reduced or lacked in the disease or the disorder. In a specific embodiment of the use of the present invention, the protein is a secretory factor.

The pharmaceutical composition of the present invention may contain one kind of mRNA or may be a mixture of a plurality of kinds of mRNAs. The pharmaceutical composition of the present invention may be a mixture of mRNAs having a variety of lengths of poly(A). The pharmaceutical composition of the present invention may contain a pharmaceutically acceptable carrier or excipient, such as a buffer solution in addition to the mRNA. The pharmaceutical composition of the present invention may contain a carrier for mRNA delivery. The pharmaceutical composition of the present invention can be administered, for example, by, but not be particularly limited to, parenteral administration. Examples of the parenteral administration include, but not be limited to, intramuscular administration, intraventricular administration, intravenous administration, intraperitoneal administration, intracerebroventricular administration, intraocular administration, subcutaneous administration, intranasal administration, intravaginal administration, and intrathecal administration. By such administration, the pharmaceutical composition can be administered in the present invention. In a certain embodiment, the pharmaceutical composition of the present invention is provided as an injection. The pharmaceutical composition of the present invention can be administered systemically or locally by an appropriate dosage form depending on the disease or the disorder.

As used herein, the term "treat" is meant to induce cure, prevention, or remission of a disease or a disorder or a reduction in rate of progress of a disease or a disorder. The treatment can be achieved by administering a therapeutically effective amount of the pharmaceutical composition.

As used herein, the term "subject" is preferably a human subject or a human patient.

In the composition or the pharmaceutical composition of the present invention, in a certain embodiment, the mRNA forms a polyion complex with PEG-PAsp(DET) in the composition. Herein, the term "PEG-PAsp(DET)" refers to a copolymer of a poly(ethylene glycol) block and a polyaspartic acid derivative block. In the PEG-PAsp(DET) used in the present invention, although the PEG has an average degree of polymerization of 5 to 20000, preferably 10 to 5000,

and more preferably 40 to 500, the degree of polymerization of the PEG is not limited as long as the formation of the polyion complex of the block copolymer and mRNA is not blocked. In the PEG-PAsp(DET) used in the present invention, in a certain embodiment, the aspartic acid derivative is aspartic acid of which the carboxyl group in the side chain is substituted with a diethyltriamine (DET) group ($-NH-CH_2-CH_2-NH-CH_2-CH_2-NH_2$). The structure of poly(Asp(DET)) is represented by the following chemical formula:

Poly(Asp(DET))

[Formula 1]

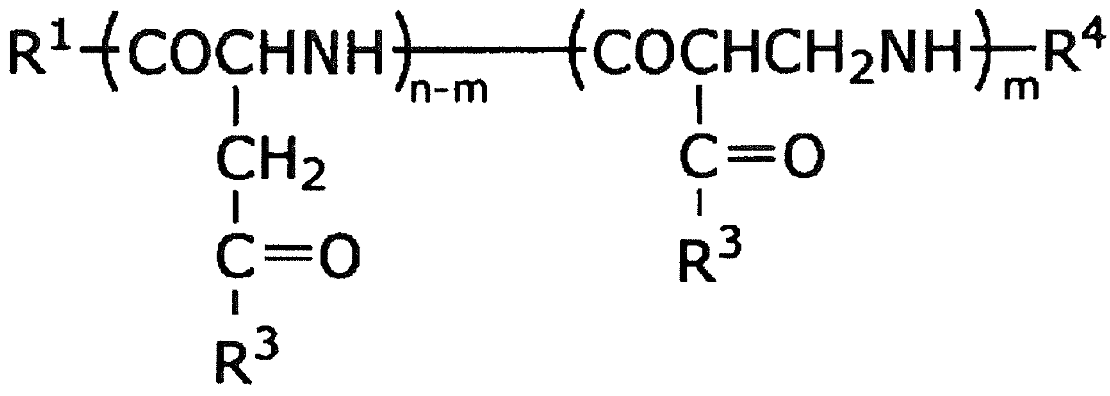

(I)

{where

$R^1$ represents a hydroxy group, a protecting group, a hydrophobic group, or a polymerizable group;
$R^4$ represents H, a protecting group, a hydrophobic group, or a polymerizable group;
$R^3$ represents a group represented by $-(NH-(CH_2)_2)_2-NH_2$ ;
n represents an integer of 0 to 5000, for example, an integer of 0 to 500;
m represents an integer of 0 to 5000, for example, an integer of 0 to 500;
m+n represents an integer of 2 to 5000, for example, an integer of 2 to 500; and
n-m represents an integer of 0 or more, and

in the formula, although the repeating units are shown in a specific order for convenience of description, the repeating units can be present in no particular order, and the repeating units may be present in random order and may be the same or different,
provided that when a polycation block forms a copolymer with poly(ethylene glycol), $R^1$ or $R^4$ represents a bond, and poly(ethylene glycol) forms a copolymer with a polycation block through the bond}. Additionally, in the polymer represented by Formula (I), the repeating units are bonded by a peptide bond.

Examples

Example 1: Construction of protein expression plasmid

In this example, plasmids having poly(A) sequences having different lengths downstream the protein-coding regions of mRNAs were constructed.

GLuc gene (derived animal species *Gaussia princeps*) and NLuc gene (derived animal species *Oplophorus gracilirostris*) were used as luciferase genes, and were cut out from pCMV-GLuc Control Plasmid (New England Biolabs Inc., Catalog No. N8081S) and pNL1.1[NLuc] Vector (Promega Corporation, Catalog No. N1001), respectively, with restriction enzymes HindIII and XbaI. Each luciferase gene was cloned between HindIII and XbaI cleavage sites in a multicloning site under the control of T7 promoter of pSP73 vector (Promega Corporation, Catalog No. P2221). The

resulting plasmid is called pSP73-Luc plasmid.

A poly(A) sequence (having a length of 120, 180, 210, 240, 270, or 360 nucleotides) was then inserted into the downstream of the luciferase gene by in-frame connection. The poly(A) tail sequence was produced using an oligo DNA, specifically, as follows.

1-1. Production of plasmid including poly(A) having a chain length of 120 nucleotides

Two oligo DNAs 5'-AATTC-$A_{121}$-GAGACGA-3' and 5'-GATCTCGTCTC-$T_{121}$-G-3' were annealed to produce a double-stranded DNA, and the double-stranded DNA was inserted into pSP73-Luc plasmid linearized using restriction enzymes EcoRI and BglII to produce a plasmid including poly(A) having a chain length of 120 nucleotides (A(120)). In the above-mentioned sequence, An represents that adenines are continuously present to form a length of n nucleotides. For example, $A_{121}$ means that adenines continue to form a length of 121 nucleotides.

1-2. Production of plasmids including poly(A) having a chain length of 180, 210, or 240 nucleotides

A double-stranded DNA produced from two oligo DNAs 5'-AATTC-$A_{120}$-GATATCA-3' and 5'-GATCTGATATC-$T_{120}$-G-3' was inserted into pSP73-Luc plasmid linearized with restriction enzymes EcoRI and BglII to produce pSP73-GLuc-A(120)-EcoRV plasmid including poly(A) having a length of 120 nucleotides and a restriction enzyme EcoRV recognition sequence. The resulting plasmid was then linearized with restriction enzymes EcoRV and BglII. Subsequently, a double-stranded DNA produced from two oligo DNAs 5'-G-$A_X$-GAGACGA-3' and 5'-GATCTCGTCTC-$T_X$-C-3' (herein, x represents 61, 91, or 121) was inserted into the restriction enzyme site of the resulting plasmid to produce plasmids including poly(A) having a chain length of 180, 210, or 240 nucleotides.

1-3. Production of plasmid including poly(A) having a chain length of 270 or 360 nucleotides

A double-stranded DNA produced from two oligo DNAs 5'-G-$A_{120}$-GATATCA-3' and 5'-GATCTGATATC-$T_{120}$-C-3' was inserted into pSP73-Luc-A(120)-EcoRV plasmid linearized using restriction enzymes EcoRV and BglII to produce pSP73-GLuc-A(240)-EcoRV plasmid. The produced plasmid was linearized using restriction enzymes EcoRV and BglII. Subsequently, a double-stranded DNA produced from two oligo DNAs 5'-G-$A_X$-GAGACGA-3' and 5'-GATCTCGTCTC-$T_X$-C-3' (herein, x represents 31 or 121) was inserted into the restriction enzyme site of the resulting plasmid to produce plasmids including poly(A) having a chain length of 270 or 360 nucleotides.

Hereinafter, the resulting plasmid is expressed as "pSP73-Luc-A (nucleotide length of poly(A))". For example, a poly(A) sequence having a length of 120 nucleotides is expressed as pSP73-Luc-A(120) in the specification.

The protein expression plasmid was replicated in *Escherichia coli* and was then purified to be used in the subsequent examples by an ordinary method in the art.

Example 2: Ability of mRNA to bind to protein

In this example, the ability of an mRNA to bind to a protein was verified.

The plasmids including poly(A) having a chain length of 120, 180, or 210 nucleotides were converted to linearized DNAs with type IIS restriction enzyme BsmBI, and the linearized DNAs were purified by agarose electrophoresis. The plasmids including poly(A) having a chain length of 240, 270, or 360 nucleotides were converted to linearized DNAs with two kinds of restriction enzymes BsmBI and HpaI, and the linearized DNAs were purified by agarose electrophoresis. The purified linearized DNAs had a luciferase gene under the control of T7 promoter. The resulting linearized DNAs were *in vitro* transcribed using mMESSAGE mMACHINE T7Ultra Kit (Ambion, Inc.) according to the manufacturer's manual to prepare mRNAs. The resulting mRNAs were purified with RNeasy Mini Kit (Qiagen N.V.). The results of agarose electrophoresis of the purified mRNAs are shown in Figure 1. Figure 1 shows the results of electrophoresis of mRNAs including A(120), A(240), and A(360).

The mRNA bound to an intranuclear protein was prepared as follows: Huh7 cells were seeded in a 96-well plate at a density of 5000 cells/well and were cultured for 24 hours. The culture medium used was a 10% FBS-containing DMEM. The GLuc mRNA including poly(A) having any of various lengths purified in Example 1 was added to each well at an amount of 190 ng to be introduced into the Huh7 cells with a gene introduction reagent Lipofectamine LTX (Life technologies, Inc.). After 24 hours from the introduction, cell lysate samples were prepared using Dynabeads Co-Immunoprecipitation Kit (Life technologies, Inc.).

The binding between an mRNA and a protein was verified by immunoprecipitation. That is, the mRNA formed a complex with a protein, PABP or eIF4E, was precipitated using an antibody against PABP or eIF4E, and the amount of the precipitated mRNA was used as an index of the binding of the mRNA to the protein. Specifically, the antibody used was an anti-PABP antibody (Abcam plc., Catalog No. ab21060) or an anti-eIF4E antibody (Santa Cruz Biotechnology, Inc., Catalog No. sc-13963). The immunoprecipitation was performed using Dynabeads Co-Immunoprecipitation Kit (Life technologies, Inc.) by incubation at 4°C for 30 minutes to bind the antibody to the protein. Subsequently, the total RNAs were collected with RNeasy Mini Kit (QIAGEN N.V.), and cDNAs were produced from the mRNAs contained in the total RNAs with RevertraAce qPCR RT Master Mix with gDNA Remover (TOYOBO Co., Ltd.).

The amount of the GLuc mRNA bound to the protein and precipitated with the antibody was quantitatively measured by a quantitative polymerase chain reaction (quantitative PCR) using ABI Prism 7500 Sequence Detector (Applied Biosystems). The primers used were forward primer: 5'-TTGAACCCAGGAATCTCAGG-3' and reverse primer: 5'-CACGCCCAAGATGAAGAAGT-3'. The results of the quantitative measurement were then standardized relative to the amount, which was defined as 1, of the mRNA having a poly(A) length of 120. The results were as shown in Figure 2.

The results of the binding of mRNAs to PABP will be described by Figure 2A. As shown in Figure 2A, the mRNA including A(240) (i.e., the poly(A) length was 240 nucleotides) significantly bound to PABP compared with the mRNA including A(120) (i.e., the poly(A) length was 120 nucleotides). The mRNA including A(360) bound to a larger amount of PABP compared with the mRNA including A(240). This demonstrated that an mRNA including long poly(A) length can advantageously bind to PABP.

The results of the binding of mRNAs to eIF4E will be then described by Figure 2B. As shown in Figure 2B, it was shown that the mRNA including A(240) bound to a significantly larger amount of eIF4E compared with the mRNAs including A(120) or A(360). It was revealed that poly(A) having a specific length is suitable for binding to eIF4E, and it was suggested that poly(A) being too long has a risk of blocking the binding to eIF4E.

Since PABP is known to bind to the poly(A) of an mRNA, the result that PABP advantageously binds to an mRNA including long poly(A) is an expected result. Since eIF4E is assumed to also bind to the poly(A) of an mRNA through PABP, it has been similarly expected that eIF4E advantageously binds to an mRNA including long poly(A). However, the results mentioned above demonstrated that eIF4E strongly binds to an mRNA including poly(A) having a specific length (specifically, an mRNA including A(240)) and significantly strongly binds to the mRNA compared with mRNAs including A(120) or A(360). That is, although an mRNA including A(360) strongly binds to PABP, the binding to eIF4E was surprisingly weakened.

Example 3: Efficiency of *in vitro* translation into protein from mRNA

In this example, the efficiency of translation of the resulting mRNA into a protein was investigated.

Huh7 cells were seeded in a 96-well plate at a density of 5000 cells/well and were cultured for 24 hours. The culture medium used was a 10% FBS-containing DMEM. The GLuc mRNA including poly(A) having any of various lengths purified in Example 1 was introduced at an amount of 190 ng into the Huh7 cells with a gene introduction reagent Lipofectamine LTX (Life technologies, Inc.). In addition, as a control, an mRNA to which poly(A) was enzymatically added using a poly(A) addition signal was prepared. The pSP73-Luc plasmid prepared in Example 1 was cleaved using a restriction enzyme NdeI. The linearized plasmid was *in vitro* translated using mMESSAGE mMACHINE T7 Ultra Kit (Ambion, Inc.) according to the manufacturer's manual to produce an mRNA. Subsequently, according to the same manufacture's manual, poly(A) was added to the resulting mRNA (reaction conditions: 37°C, 45 min). The resulting mRNA was purified with RNeasy Mini Kit (Qiagen N.V.) and was introduced into the Huh7 cells as described above.

The quantitative measurement was performed based on the amount of luminescence from the luciferase. Specifically, the amount of luciferase luminescence from each mRNA was measured using Renilla Luciferase assay System (Promega Corporation) with GloMaxTM 96 microplate luminometer (Promega Corporation). The amount of luminescence was determined by relative luciferase units (RLU). The results were as shown in Figure 3.

As shown in Figure 3, the mRNAs having A(120), A(180), or A(210) showed approximately the same degree of protein expression. Compared with these expression levels, the mRNA having A(240) showed a significantly high level of protein expression. This increase in expression level was statistically significant. In contrast, the protein expression of the mRNAs having A(270) or A(360) was statistically significantly lower compared with that of the mRNA having A(240). These results revealed that the protein expression when the poly(A) length is 240 is significantly increased compared with those when the poly(A) length is 210 or 270. The significant decrease in the translation efficiency of the mRNA having A(270) suggested that poly(A) of 270 or more nucleotides gained an activity of blocking the translation.

The mRNA having A(90) showed protein expression equivalent to the level of the mRNA having A(120) (data not shown). In addition, when luciferase is expressed using a protein expression plasmid including a poly(A) addition signal (AATAAA) (the above-mentioned control) instead of poly(A), the RLU was only the same as that of the mRNA having A(360). That is, the expression level of a target protein by the mRNA having A(240) was much higher than the expression level by an mRNA to which poly(A) was enzymatically added, which is similar to a natural mRNA.

A(180), A(210), A(240), A(270), and A(360) each contain an intervening sequence (GAUG sequence) derived from a restriction enzyme site between poly(A) and poly(A). In order verify the influence of GAUG on protein expression, A(120) and A(60)-GAUG-A(60) were produced as in above, and the expression levels were compared. The expression level of A(60)-GAUG-A(60) was about 80% of that of A(120), which revealed that the influence of the intervening sequence

is limited (data not shown).

The same results were observed in cell-free translation systems (rabbit reticulocyte lysate and human cell-free extract). Specifically, Rabbit Reticulocyte Lysate, Untreated (Promega Corporation) was used as the rabbit reticulocyte lysate, and Human Cell-Free Protein Expression System (Takara Bio Inc.) was used as the human cell-free extract. GLuc mRNAs having each poly(A) length were incubated at 30°C for 120 minutes or at 32°C for 30 minutes. The expression levels were quantitatively measured based on the amounts of luminescence from luciferase. The amount of luciferase luminescence from each mRNA was measured using Renilla Luciferase assay System (Promega Corporation) with GloMaxTM 96 microplate luminometer (Promega Corporation).

The results were as shown in Figure 4. As shown in Figures 4A and 4B, the protein expression level of the mRNA having A(240) was significantly higher than those of the mRNAs having A(120) or A(360). It was therefore confirmed that even if a cell-free extract system is used, the expression level of an mRNA including poly(A) having a length of 240 nucleotides is significantly high compared with mRNAs including poly(A) having the other lengths.

Example 4: Efficiency of *in vivo* translation from mRNA into protein

In this example, the expression efficiency of luciferase protein in mouse skeletal muscle was investigated.

NLuc mRNAs having A(120), A(240), or A(360) prepared in Example 1 were used. Administration was performed using a nano-micelle type carrier (see PLoS One 8(2): e56220, 2013) containing an mRNA. Specifically, the block copolymer used for constituting the nano-micelle was PEG-PAsp(DET) including a PEG block portion having a number-average molecular weight of 12000 and an Asp(DET) block portion having a number-average degree of polymerization of 65. The PEG-PAsp(DET) is a copolymer of a poly(ethylene glycol) block and a polyaspartic acid derivative block. The aspartic acid derivative is aspartic acid of which the carboxyl group is substituted with a diethyltriamine group ($-NH-CH_2-CH_2-NH-CH_2-CH_2-NH_2$). The PEG-PAsp(DET) and the mRNA were respectively dissolved in a 10 mM HEPES buffer, and the both were mixed to prepare a nano-micelle solution. The nano-micelle solution was prepared such that the molar ratio, N/P ratio, of the amino group (N) of the PEG-PAsp(DET) to the phosphate group (P) of the mRNA was 8.

The micelle was administered into the lower limb skeletal muscle of a mouse by hydrodynamics method. Specifically, the mouse was anesthetized with 3% isoflurane (Abbott Japan Co., Ltd.), and a tourniquet was then indwelled at the proximal femur to temporarily block the blood circulation in the lower limb. Subsequently, 300 μL of the nano-micelle solution containing 5 μg of an mRNA was administered from the great saphenous vein behind the ankle medial malleolus over 5 seconds. After 5 minutes from the administration, the tourniquet was removed.

Subsequently, the amount of the luciferase protein expressed in the skeletal muscle after 72 hours from the administration was quantitatively measured. Specifically, the lower limb skeletal muscle after 72 hours from the administration was collected, and the tissue was homogenized with Multi-beads shocker (Yasui Kikai Corporation). The amount of the luciferase protein was quantitatively measured based on the amount of luciferase luminescence. The quantitative measurement of the amount of luminescence was carried out using Nano-GLo Luciferase assay system (Promega Corporation) and Lumat LB9507 luminometer (Berthold Technologies), and the amount of luminescence was standardized by the protein concentration in the cell lysate.

As a result, as shown in Figure 4, the mRNA including A(240) showed protein expression at a significantly high level compared with the mRNA including A(120) or A(360).

These results revealed that in order to enhance the translation efficiency of an mRNA, it is preferable to adjust the length of poly(A) to about 240 nucleotides.

Example 5: Treatment of sciatic nerve damage model mouse

In this example, IGF-1-expressing mRNA molecules including poly(A) having different lengths were administered to sciatic nerve damage model mice to verify the therapeutic effect.

The sciatic nerve of the left leg of each mouse (Balb/c albino mouse, female, 10 to 14 week-old, purchased from Charles River Laboratories) was exposed near the greater trochanter, and the exposed sciatic nerve was pressed with tweezers cooled with liquid nitrogen to prepare a sciatic nerve damage model mouse.

Poly (A) having a length of 120 nucleotides or 240 nucleotides was added to the 3'UTR region of an IGF-1-expressing mRNA, and the mRNA was mixed with a PEG-poly(N'-[N-(2-aminoethyl)-2-aminoethyl]-aspartic acid) block copolymer (PEG-PAsp(DET)) to form a polyion complex micelle.

The PEG-PAsp(DET) was prepared according to an ordinary method (see ChemMedChem, 1 (2006), 439-444). It was estimated by $H^1$-NMR measurement that the PEG portion had a number-average molecular weight of 12000 and the PAsp(DET) portion had a degree of polymerization of 69. The resulting PEG-PAsp(DET) block copolymer and the mRNA were each dissolved in 10 mM Tris-HCl (pH 7.4), and the resulting solutions were mixed with each other to form a polyion complex micelle for administration.

Avascularization was performed near the femur of the mouse, and the resulting micelle was intravenously

administered to the leg of the disease model to allow the micelle to permeate into the muscular tissue. In order verify the therapeutic effect, the motor function of the mouse was evaluated for the period from the 7th to the 28th day after the administration. As a negative control, the motor function of a sciatic nerve damage model mouse administered with an mRNA for luciferase, instead of IGF-1, was evaluated.

The evaluation of the motor function was performed by obtaining footprints of a freely walked mouse as shown in Figure 6A and using the Sciatic Functional Index (SFI), which is known as an index for evaluating recovery of motor function, as an index (regarding the SFI, see Inserra MM, et al., Microsurgery, 1998, 18(2): 119-124). The SFI was calculated based on the following expression:

[Expression 1]

$$SFI = -51.2\left(\frac{EPL - NPL}{NPL}\right) + 118.9\left(\frac{EPW - NPW}{NPW}\right) - 7.5$$

{where, EPL represents the length in the walking direction of the footprint of the leg on the disease model side; NPL represents the length in the walking direction of the footprint of the healthy leg; the EPW represents the width of the footprint of the leg on the disease model side; and NPW represents the width of the footprint of the healthy leg}.

In the acquisition of the footprints, a walking analyzer CatWalk (manufactured by Noldus Inc.) was used. EPL, NPL, EPW, and NPW are the same as those described in the above-mentioned expression and are also shown in Figure 6B. Ideally, SFI is -100 when a leg is completely paralyzed and is 0 when a leg is normal, and an increase in the SFI value means recovery of motor function of the leg.

An IGF-1-expressing mRNA was administered to the leg on the disease model side of the sciatic nerve damage model mouse, and the walking recovery of the mouse on the 7th, 10th, 12th, 14th, 21st, and 28th days after the administration was investigated using the SFI value as the index. The results were as shown in Figure 6C.

As shown in Figure 6C, although the motor function recovered with time even in the negative control, significant recovery of the motor function was observed in the case of the poly(A) having a length of 240 (IGF-1 240A). In addition, considerable recovery of the motor function was observed in the case of the poly(A) having a length of 240 (IGF-1 240A), compared to the case of the poly(A) having a length of 120 (IGF-1 120A).

Thus, the expression level of the protein from an mRNA is significantly increased by adjusting the poly(A) of the mRNA to a certain length (in particular, a length of 240 nucleotides), and this effect was confirmed in vivo.

## Claims

**1.** A composition for use in expressing a target protein, the composition comprising:

an mRNA encoding the target protein, wherein
80% or more of the mRNA molecules contained in the composition have a sequence consisting substantially of poly(A) having a length of 230 to 250 nucleotides on the 3'-end side of the protein-coding region thereof.

**2.** The composition according to claim 1, wherein 90% or more of the mRNA molecules contained in the composition have a sequence consisting substantially of poly(A) having a length of 230 to 250 nucleotides on the 3'-end side of the protein-coding region thereof.

**3.** The composition according to claim 1, wherein 95% or more of the mRNA molecules contained in the composition have a sequence consisting substantially of poly(A) having a length of 230 to 250 nucleotides on the 3'-end side of the protein-coding region thereof.

**4.** The composition according to any one of claims 1 to 3, wherein 20% or less of the mRNA molecules contained in the composition have poly(A) having a length of 270 or more nucleotides.

**5.** A protein expression vector comprising:

a gene encoding a protein and operably linked to a promoter; and
a sequence consisting substantially of poly(A) having a length of 230 to 250 nucleotides downstream the protein-coding region of the gene encoding the protein.

**6.** A method for improving an ability of an mRNA to bind to eIF4E, comprising:

adding a sequence consisting of poly(A) or consisting substantially of poly(A) to the downstream side of the protein-coding region of a DNA to be transcribed into the mRNA such that the mRNA has a sequence consisting substantially of poly(A) having a length of 230 to 250 nucleotides on the 3'-end side of the protein-coding region thereof.

**7.** A method for expressing a target protein in a cell in the body of a subject, comprising:

providing a composition comprising an mRNA encoding the target protein, where 80% or more of the mRNA molecules contained in the composition have a sequence consisting substantially of a poly(A) sequence having a length of 230 to 250 nucleotides on the 3'-end side of the protein-coding region of the mRNA; and
administering the composition to the subject.

**8.** A pharmaceutical composition for use in treating a disease or a disorder in a subject suffering from the disease or a subject having the disorder, the pharmaceutical composition comprising:

an mRNA encoding a protein that can treat the disease or the disorder, wherein 80% or more of the mRNA molecules contained in the composition have a sequence consisting substantially of a poly(A) sequence having a length of 230 to 250 nucleotides on the 3'-end side of the protein-coding region of the mRNA.

**9.** The composition according to claim 8, wherein the disease or the disorder is a disease or a disorder caused by a reduction or a lack of a protein, and the protein that can treat the disease or the disorder is the protein that is reduced or lacked in the subject.

**10.** The composition according to claim 8, wherein the disease or the disorder is spinal cord injury, and the protein that can treat the disease or the disorder is brain-derived neurotrophic factor (BDNF).

**11.** The composition according to claim 8, wherein the disease or the disorder is peripheral nerve injury, and the protein that can treat the disease or the disorder is insulin-like growth factor (IGF-1).

FIG.1

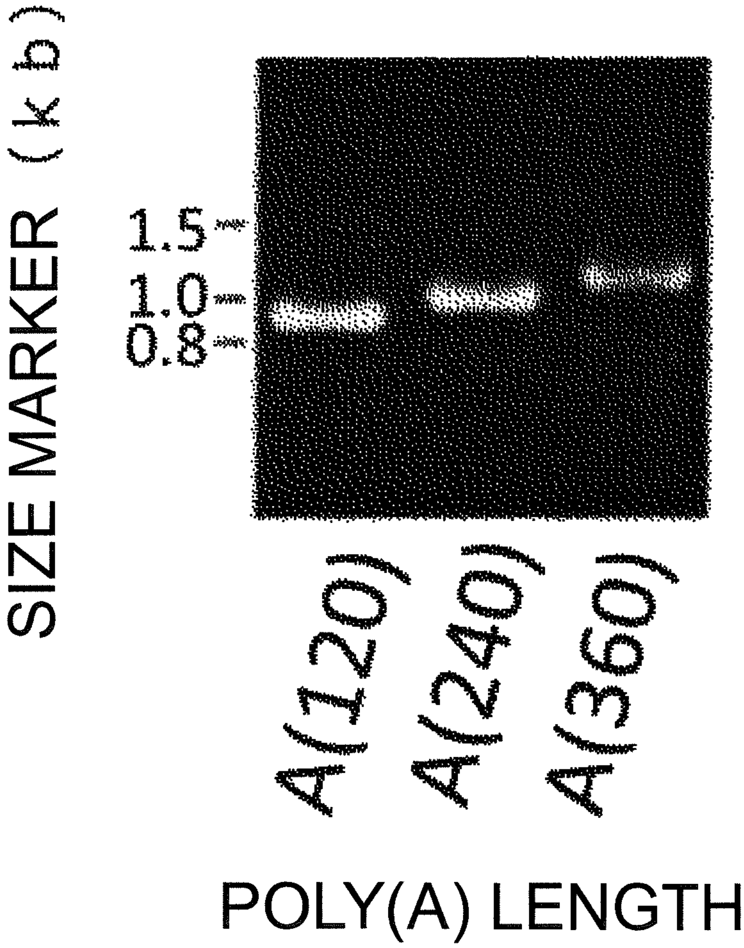

FIG.2

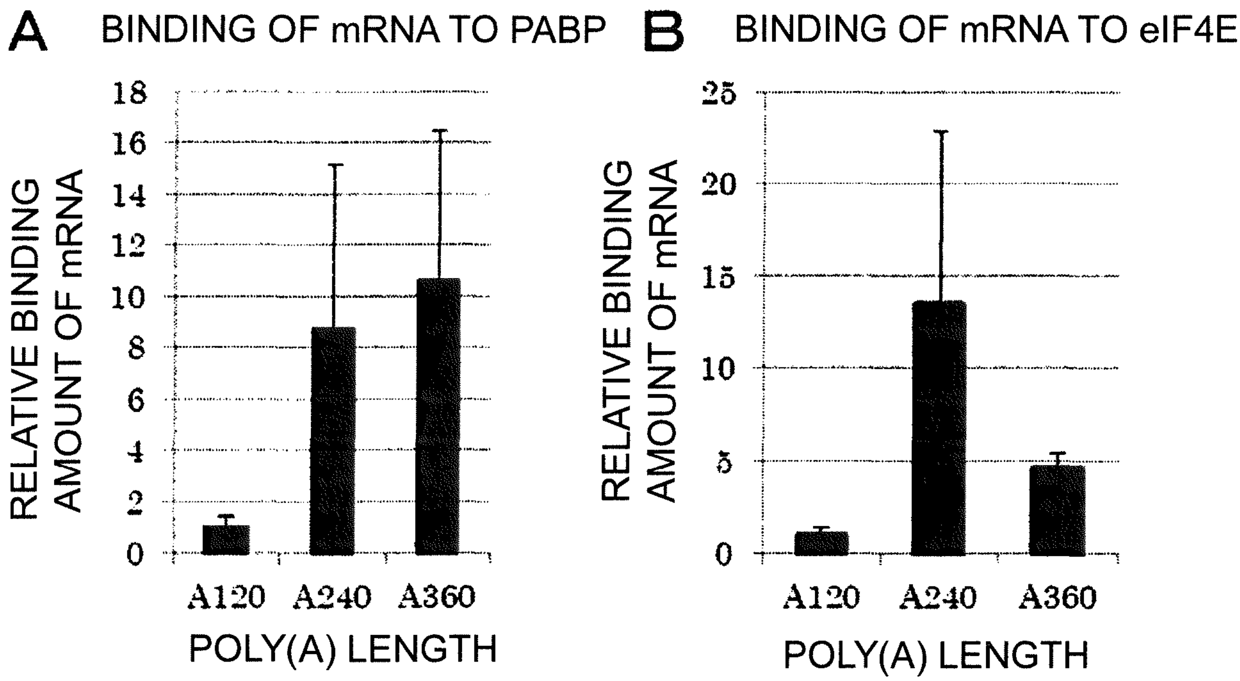
A
BINDING OF mRNA TO PABP
RELATIVE BINDING AMOUNT OF mRNA
18
16
14
12
10
8
6
4
2
0
A120
A240
A360
POLY(A) LENGTH
B
BINDING OF mRNA TO eIF4E
RELATIVE BINDING AMOUNT OF mRNA
25
20
15
10
5
0
A120
A240
A360
POLY(A) LENGTH

FIG.3

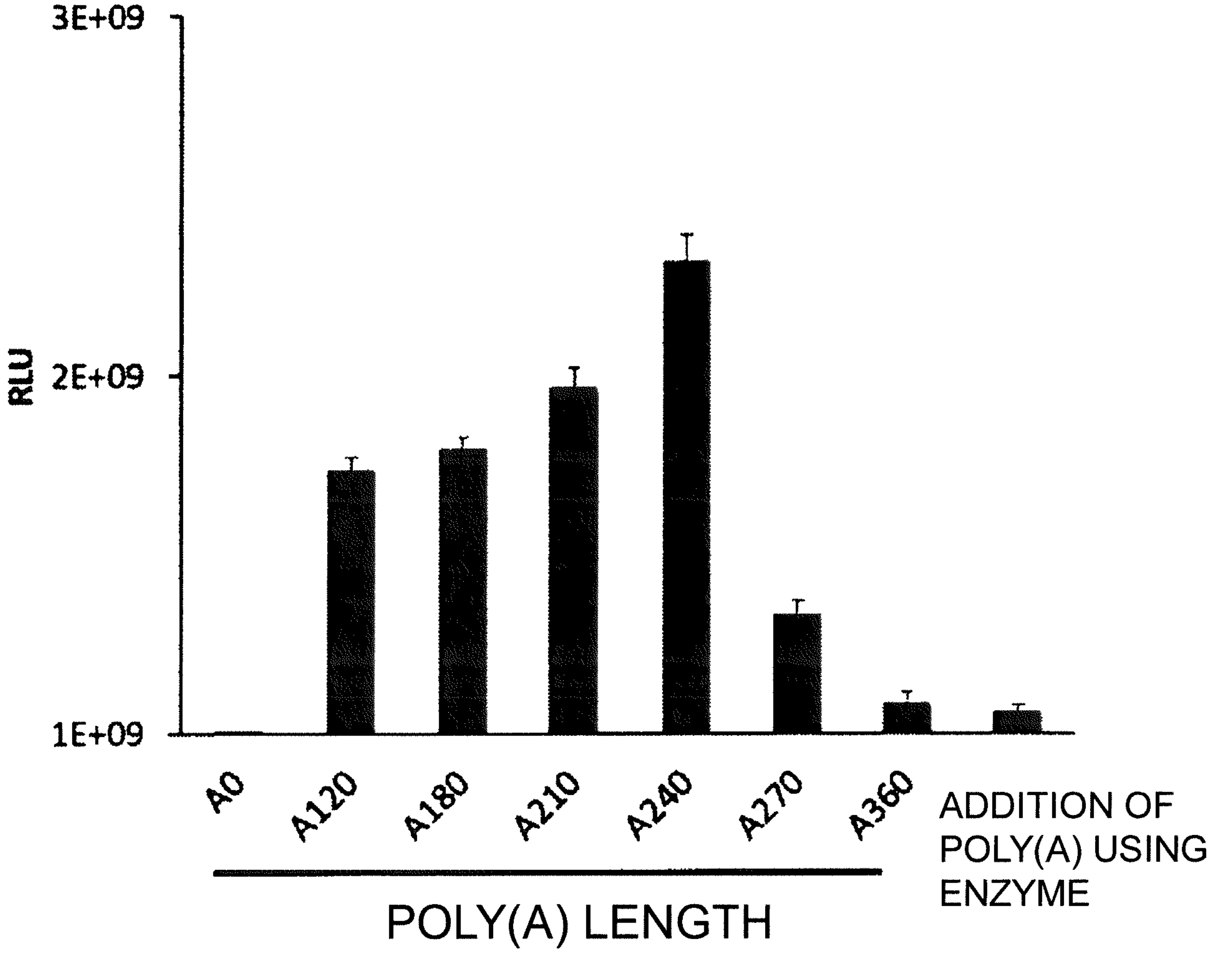
3E+09
2E+09
1E+09
RLU
A0
A120
A180
A210
A240
A270
A360
ADDITION OF POLY(A) USING ENZYME
POLY(A) LENGTH

FIG.4

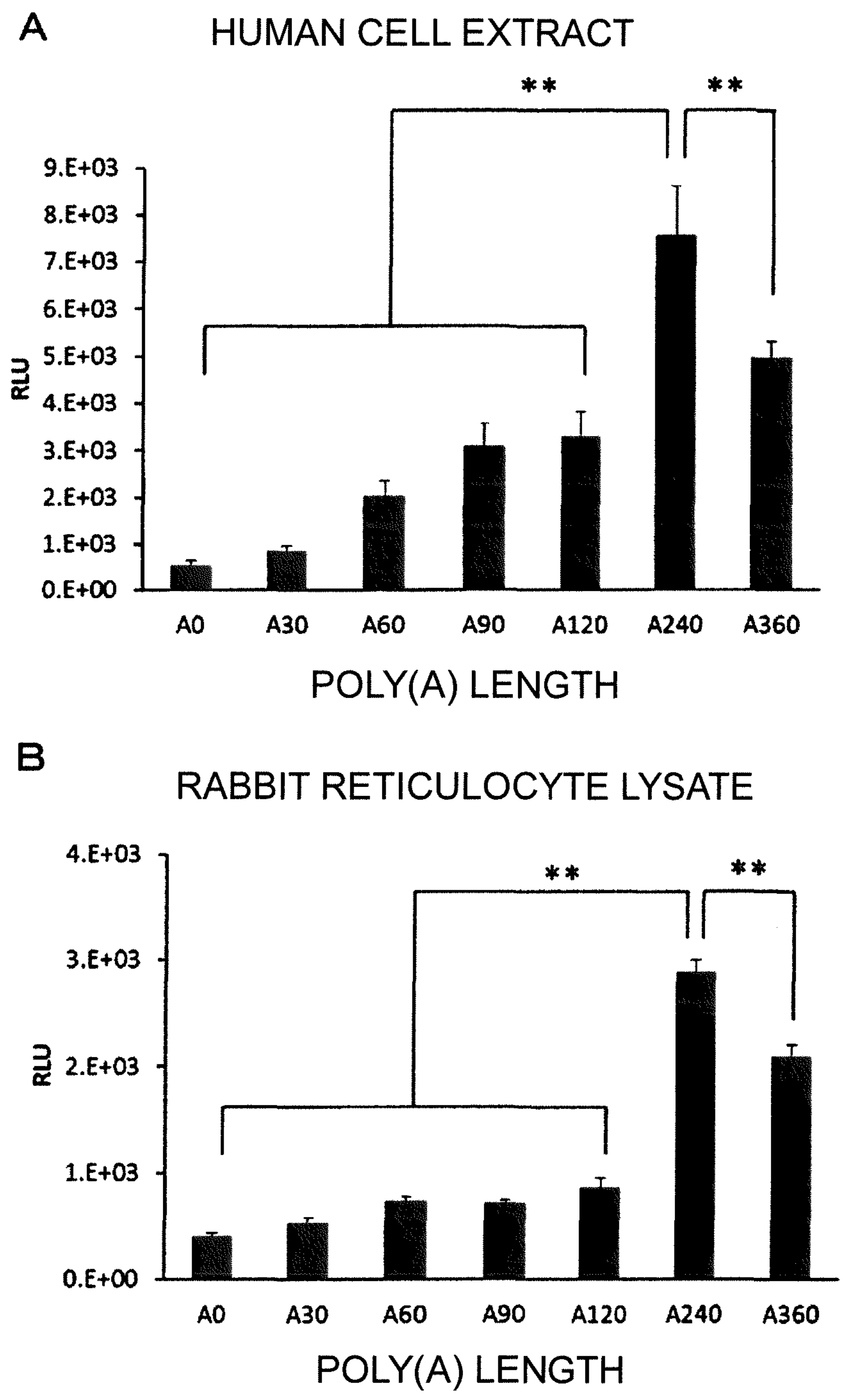
A
HUMAN CELL EXTRACT
**
**
9.E+03
8.E+03
7.E+03
6.E+03
5.E+03
4.E+03
3.E+03
2.E+03
1.E+03
0.E+00
RLU
A0
A30
A60
A90
A120
A240
A360
POLY(A) LENGTH
B
RABBIT RETICULOCYTE LYSATE
4.E+03
3.E+03
2.E+03
1.E+03
0.E+00
RLU
**
**
A0
A30
A60
A90
A120
A240
A360
POLY(A) LENGTH

FIG.5

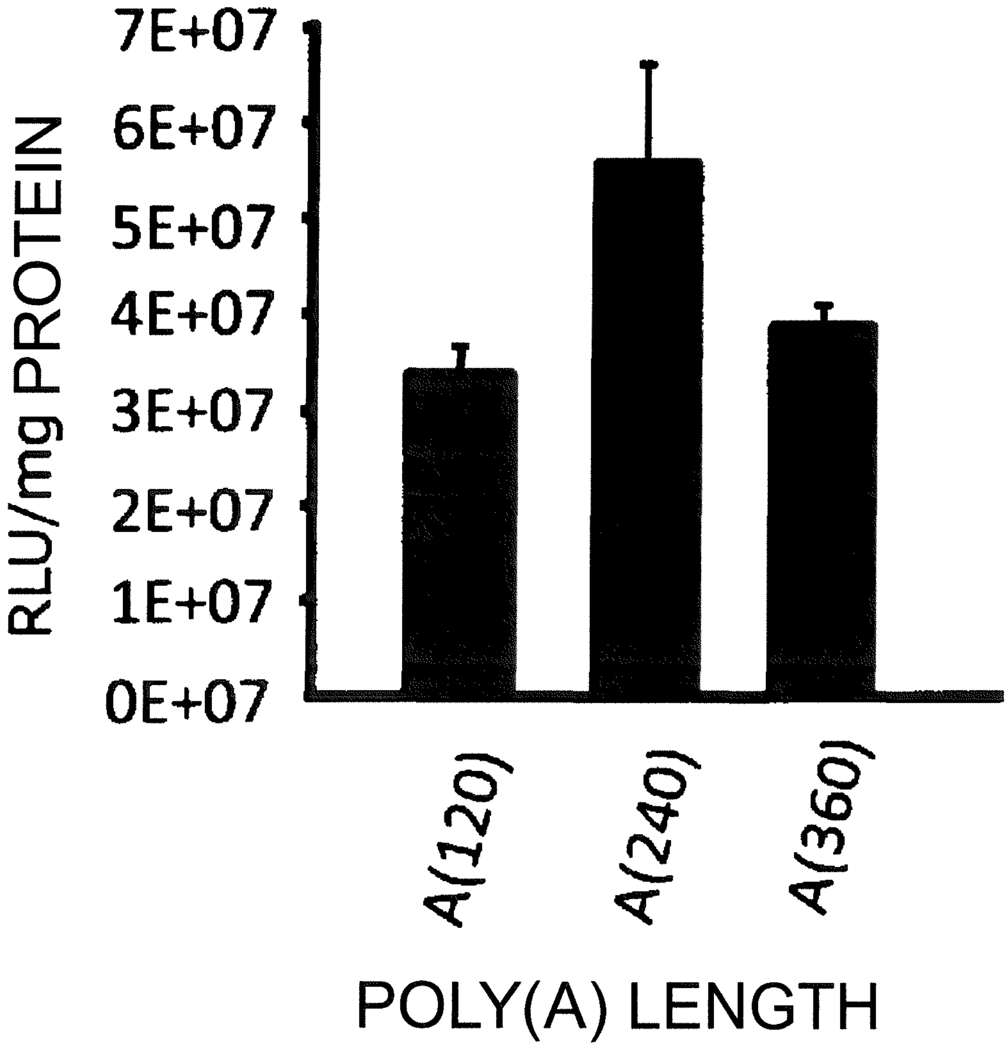
7E+07
6E+07
5E+07
4E+07
3E+07
2E+07
1E+07
0E+07
RLU/mg PROTEIN
A(120)
A(240)
A(360)
POLY(A) LENGTH

FIG.6

A

ACQUISITION OF FOOTPRINT OF FREELY WALKED MOUSE

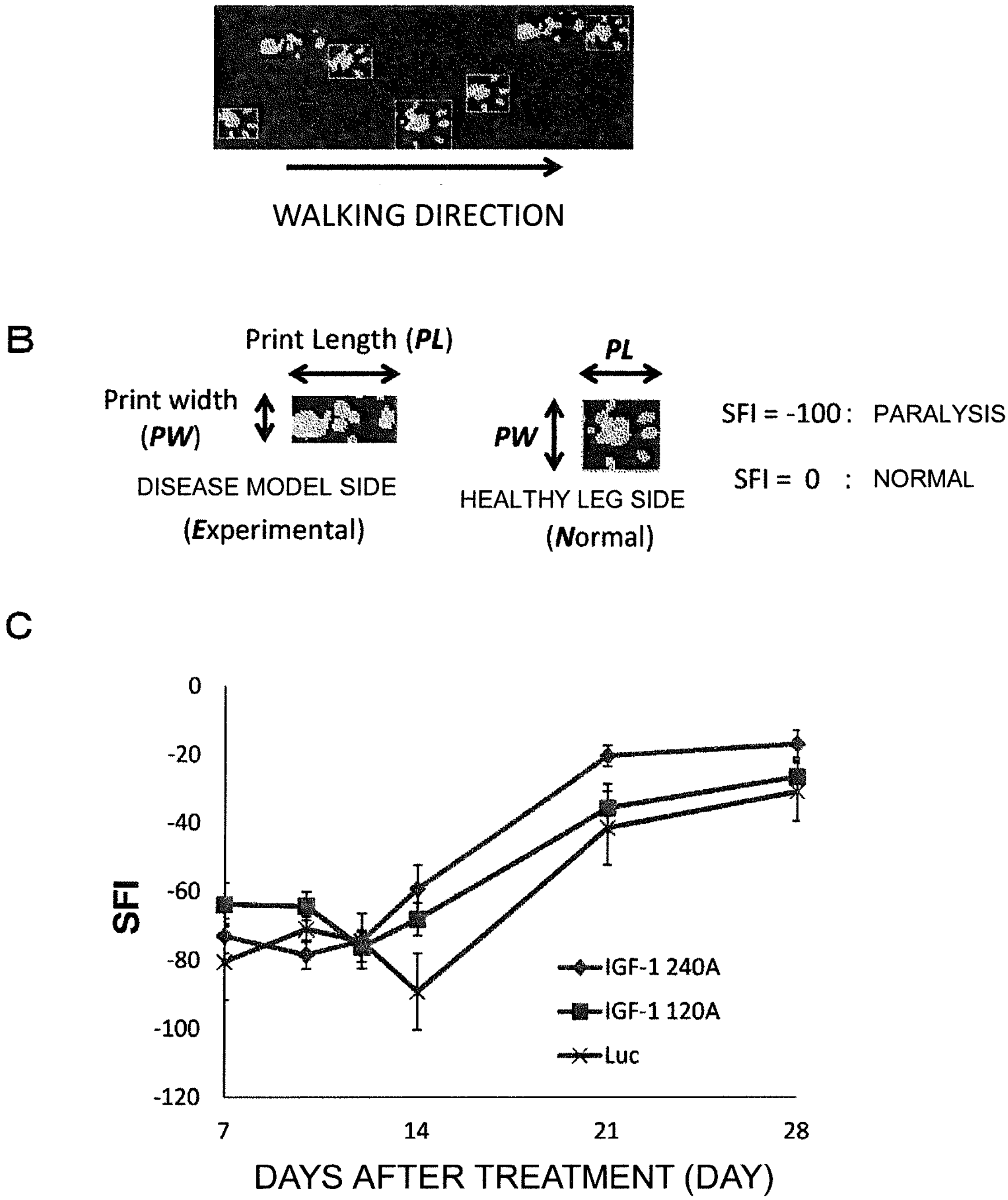

## INTERNATIONAL SEARCH REPORT

International application No.
PCT/JP2015/002208

A. CLASSIFICATION OF SUBJECT MATTER
*C12N15/09*(2006.01)i, *A61K31/7105*(2006.01)i, *A61K48/00*(2006.01)i, *A61P25/00*(2006.01)i, *A61P25/02*(2006.01)i, *A61P43/00*(2006.01)i

According to International Patent Classification (IPC) or to both national classification and IPC

B. FIELDS SEARCHED

Minimum documentation searched (classification system followed by classification symbols)
C12N15/09, A61K31/7105, A61K48/00, A61P25/00, A61P25/02, A61P43/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)
JSTPlus/JMEDPlus/JST7580(JDreamIII), CAplus/MEDLINE/EMBASE/BIOSIS(STN)

C. DOCUMENTS CONSIDERED TO BE RELEVANT

| Category* | Citation of document, with indication, where appropriate, of the relevant passages | Relevant to claim No. |
|---|---|---|
| A | JP 2002-508299 A (Sequitur, Inc.), 19 March 2002 (19.03.2002), & US 2003/0083272 A1 & WO 1999/014346 A2 & EP 1021549 A | 1-6,8-11 |
| A | JP 2010-508014 A (CureVac GmbH), 18 March 2010 (18.03.2010), & US 2010/0047261 A1 & WO 2008/052770 A2 & EP 2083851 A | 1-6,8-11 |
| A | JP 2006-517802 A (Biogen Idec MA Inc.), 03 August 2006 (03.08.2006), & US 2006/0141625 A1 & WO 2004/074439 A2 & EP 1594955 A | 1-6,8-11 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 23 July 2015 (23.07.15) | 04 August 2015 (04.08.15) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (July 2009)

**INTERNATIONAL SEARCH REPORT**

International application No.
PCT/JP2015/002208

C (Continuation). DOCUMENTS CONSIDERED TO BE RELEVANT

| Category* | Citation of document, with indication, where appropriate, of the relevant passages | Relevant to claim No. |
|---|---|---|
| A | BECKEL-MITCHENER A. C. et al., Poly(A) Tail Length-dependent Stabilization of GAP-43 mRNA by theRNA-binding Protein HuD, J. Biol. Chem., 2002, vol.277, no.31, p.27996-8002 | 1-6,8-11 |
| A | BEILHARZ T. H. et al., Widespread use of poly(A) tail length control to accentuate expression of the yeast transcriptome, RNA, 2007, vol.13, p.982-97 | 1-6,8-11 |
| A | HOLTKAMP S. et al., Modification of antigen-encoding RNA increases stability, translational efficacy, and T-cell stimulatory capacity of dendritic cells, Blood, 2006, vol.108, no.13, p.4009-17 | 1-6,8-11 |

Form PCT/ISA/210 (continuation of second sheet) (July 2009)

INTERNATIONAL SEARCH REPORT

International application No.
PCT/JP2015/002208

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 7
because they relate to subject matter not required to be searched by this Authority, namely:
The claim involves a method for therapy of a human being.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2014090634 A


### Non-patent literature cited in the description


- **E. WAHLE.** *Journal of Biological Chemistry,* 1995, vol. 270, 2800-2808
- **HOLTKAMP et al.** *Blood,* 2006, vol. 108, 4009-4017
- *PLoS One,* 2013, vol. 8 (2), e56220
- *ChemMedChem,* 2006, vol. 1, 439-444
- **INSERRA MM et al.** *Microsurgery,* 1998, vol. 18 (2), 119-124

SEQUENCE LISTING

<110> Medical Industry Innovation Institute

<120> METHOD FOR IMPROVING PROTEIN EXPRESSION AND COMPOSITION USED THEREFOR

<130> PI38-9002WO

<150> JP 2014-090634
<151> 2014-04-24

<160> 25

<170> PatentIn version 3.5

<210> 1
<211> 3048
<212> DNA
<213> Artificial Sequence

<220>
<223> pSP73-Luc

<400> 1
gaaccagatc tgatatcatc gatgaattcg agctcggtac ccggggatcc tctagatgca 60

tgctcgagcg gccgcttagt caccaccggc cccttgatc ttgtccacct ggccctggat 120

cttgctggca aaggtcgcac agcgttgcgg cagccacttc ttgagcaggt cagaacactg 180

cacgttggca agccctttga ggcagccagt tgtgcagtcc acacacagat cgacctgtgc 240

gatgaactgc tccatgggct ccaagtcctt gaacccagga atctcaggaa tgtcgacgat 300

cgcctcgcct atgccgccct gtgcggactc tttgtcgcct tcgtaggtgt ggcagcgtcc 360

tgggatgaac ttcttcatct tgggcgtgca cttgatgtgg gacaggcaga tcagacagcc 420

cctggtgcag ccagctttcc gggcattggc ttccatctct ttgagcacct ccagcggcag 480

cttcttgccg ggcaacttcc cgcggtcagc atcgagatcc gtggtcgcga agttgctggc 540

cacggccacg atgttgaagt cttcgttgtt ctcggtgggc ttggcctcgg ccacagcgat 600

gcagatcagg gcaaacagaa ctttgactcc catggtggct ggatccgagc tcggtaccaa 660

gcttcagctg ctcgaggccg gtctccctat agtgagtcgt attaatttcg ataagccagg 720

ttaacctgca ttaatgaatc ggccaacgcg cggggagagg cggtttgcgt attgggcgct 780

cttccgcttc ctcgctcact gactcgctgc gctcggtcgt tcggctgcgg cgagcggtat 840

cagctcactc aaaggcggta atacggttat ccacagaatc aggggataac gcaggaaaga 900

acatgtgagc aaaaggccag caaaaggcca ggaaccgtaa aaaggccgcg ttgctggcgt 960

ttttccatag gctccgcccc cctgacgagc atcacaaaaa tcgacgctca agtcagaggt 1020

ggcgaaaccc gacaggacta taaagatacc aggcgtttcc ccctggaagc tccctcgtgc 1080

gctctcctgt tccgaccctg ccgcttaccg gatacctgtc cgcctttctc ccttcgggaa 1140

gcgtggcgct ttctcatagc tcacgctgta ggtatctcag ttcggtgtag gtcgttcgct 1200
ccaagctggg ctgtgtgcac gaaccccccg ttcagcccga ccgctgcgcc ttatccggta 1260
actatcgtct tgagtccaac ccggtaagac acgacttatc gccactggca gcagccactg 1320
gtaacaggat tagcagagcg aggtatgtag gcggtgctac agagttcttg aagtggtggc 1380
ctaactacgg ctacactaga agaacagtat ttggtatctg cgctctgctg aagccagtta 1440
ccttcggaaa aagagttggt agctcttgat ccggcaaaca aaccaccgct ggtagcggtg 1500
gtttttttgt ttgcaagcag cagattacgc gcagaaaaaa aggatctcaa gaagatcctt 1560
tgatcttttc tacggggtct gacgctcagt ggaacgaaaa ctcacgttaa gggattttgg 1620
tcatgagatt atcaaaaagg atcttcacct agatcctttt aaattaaaaa tgaagtttta 1680
aatcaatcta aagtatatat gagtaaactt ggtctgacag ttaccaatgc ttaatcagtg 1740
aggcacctat ctcagcgatc tgtctatttc gttcatccat agttgcctga ctccccgtcg 1800
tgtagataac tacgatacgg gagggcttac catctggccc cagtgctgca atgataccgc 1860
gagacccacg ctcaccggct ccagatttat cagcaataaa ccagccagcc ggaagggccg 1920
agcgcagaag tggtcctgca actttatccg cctccatcca gtctattaat tgttgccggg 1980
aagctagagt aagtagttcg ccagttaata gtttgcgcaa cgttgttgcc attgctacag 2040
gcatcgtggt gtcacgctcg tcgtttggta tggcttcatt cagctccggt tcccaacgat 2100
caaggcgagt tacatgatcc cccatgttgt gcaaaaaagc ggttagctcc ttcggtcctc 2160
cgatcgttgt cagaagtaag ttggccgcag tgttatcact catggttatg gcagcactgc 2220
ataattctct tactgtcatg ccatccgtaa gatgcttttc tgtgactggt gagtactcaa 2280
ccaagtcatt ctgagaatag tgtatgcggc gaccgagttg ctcttgcccg gcgtcaatac 2340
gggataatac cgcgccacat agcagaactt taaaagtgct catcattgga aaacgttctt 2400
cggggcgaaa actctcaagg atcttaccgc tgttgagatc cagttcgatg taacccactc 2460
gtgcacccaa ctgatcttca gcatctttta ctttcaccag cgtttctggg tgagcaaaaa 2520
caggaaggca aaatgccgca aaaaagggaa taagggcgac acggaaatgt tgaatactca 2580
tactcttcct ttttcaatat tattgaagca tttatcaggg ttattgtctc atgagcggat 2640
acatatttga atgtatttag aaaaataaac aaataggggt tccgcgcaca tttccccgaa 2700
aagtgccacc tgacgtctaa gaaaccatta ttatcatgac attaacctat aaaaataggc 2760
gtatcacgag gccctttcgt ctcgcgcgtt tcggtgatga cggtgaaaac ctctgacaca 2820
tgcagctccc ggagacggtc acagcttgtc tgtaagcgga tgccgggagc agacaagccc 2880
gtcagggcgc gtcagcgggt gttggcgggt gtcggggctg gcttaactat gcggcatcag 2940
agcagattgt actgagagtg caccatatgg acatattgtc gttagaacgc ggctacaatt 3000
aatacataac cttatgtatc atacacatac gatttaggtg acactata 3048

<210> 2
<211> 133
<212> DNA
<213> Artificial Sequence

<220>
<223> 5′-AATTC-A121-GAGACGA-3′

<400> 2
aattcaaaaa aaaaaaaaaa aaaaaaaaaa aaaaaaaaaa aaaaaaaaaa aaaaaaaaaa 60

aaaaaaaaaa aaaaaaaaaa aaaaaaaaaa aaaaaaaaaa aaaaaaaaaa aaaaaaaaaa 120

aaaaaagaga cga 133

<210> 3
<211> 133
<212> DNA
<213> Artificial Sequence

<220>
<223> 5′-GATCTCGTCTC-T121-G-3′

<400> 3
gatctcgtct cttttttttt tttttttttt tttttttttt tttttttttt tttttttttt 60

tttttttttt tttttttttt tttttttttt tttttttttt tttttttttt tttttttttt 120

tttttttttt ttg 133

<210> 4
<211> 3163
<212> DNA
<213> Artificial Sequence

<220>
<223> pSP73-Luc+T121

<400> 4
gaaccagatc tcgtctcttt tttttttttt tttttttttt tttttttttt tttttttttt 60

tttttttttt tttttttttt tttttttttt tttttttttt tttttttttt tttttttttt 120

tttttttttt ttttttttga attcgagctc ggtacccggg gatcctctag atgcatgctc 180

gagcggccgc ttagtcacca ccggcccct tgatcttgtc cacctggccc tggatcttgc 240

tggcaaaggt cgcacagcgt tgcggcagcc acttcttgag caggtcagaa cactgcacgt 300

tggcaagccc tttgaggcag ccagttgtgc agtccacaca cagatcgacc tgtgcgatga 360

actgctccat gggctccaag tccttgaacc caggaatctc aggaatgtcg acgatcgcct 420

cgcctatgcc gccctgtgcg gactctttgt cgccttcgta ggtgtggcag cgtcctggga 480

tgaacttctt catcttgggc gtgcacttga tgtgggacag gcagatcaga cagcccctgg 540

tgcagccagc tttccgggca ttggcttcca tctctttgag cacctccagc ggcagcttct 600

tgccgggcaa cttcccgcgg tcagcatcga gatccgtggt cgcgaagttg ctggccacgg 660
ccacgatgtt gaagtcttcg ttgttctcgg tgggcttggc ctcggccaca gcgatgcaga 720
tcagggcaaa cagaactttg actcccatgg tggctggatc cgagctcggt accaagcttc 780
agctgctcga ggccggtctc cctatagtga gtcgtattaa tttcgataag ccaggttaac 840
ctgcattaat gaatcggcca acgcgcgggg agaggcggtt tgcgtattgg gcgctcttcc 900
gcttcctcgc tcactgactc gctgcgctcg gtcgttcggc tgcggcgagc ggtatcagct 960
cactcaaagg cggtaatacg gttatccaca gaatcagggg ataacgcagg aaagaacatg 1020
tgagcaaaag gccagcaaaa ggccaggaac cgtaaaaagg ccgcgttgct ggcgtttttc 1080
cataggctcc gcccccctga cgagcatcac aaaaatcgac gctcaagtca gaggtggcga 1140
aacccgacag gactataaag ataccaggcg tttccccctg gaagctccct cgtgcgctct 1200
cctgttccga ccctgccgct taccggatac ctgtccgcct ttctcccttc gggaagcgtg 1260
gcgctttctc atagctcacg ctgtaggtat ctcagttcgg tgtaggtcgt tcgctccaag 1320
ctgggctgtg tgcacgaacc ccccgttcag cccgaccgct gcgccttatc cggtaactat 1380
cgtcttgagt ccaacccggt aagacacgac ttatcgccac tggcagcagc cactggtaac 1440
aggattagca gagcgaggta tgtaggcggt gctacagagt tcttgaagtg gtggcctaac 1500
tacggctaca ctagaagaac agtatttggt atctgcgctc tgctgaagcc agttaccttc 1560
ggaaaaagag ttggtagctc ttgatccggc aaacaaacca ccgctggtag cggtggtttt 1620
tttgtttgca agcagcagat tacgcgcaga aaaaaaggat ctcaagaaga tcctttgatc 1680
ttttctacgg ggtctgacgc tcagtggaac gaaaactcac gttaagggat tttggtcatg 1740
agattatcaa aaaggatctt cacctagatc cttttaaatt aaaaatgaag ttttaaatca 1800
atctaaagta tatatgagta aacttggtct gacagttacc aatgcttaat cagtgaggca 1860
cctatctcag cgatctgtct atttcgttca tccatagttg cctgactccc cgtcgtgtag 1920
ataactacga tacgggaggg cttaccatct ggccccagtg ctgcaatgat accgcgagac 1980
ccacgctcac cggctccaga tttatcagca ataaaccagc cagccggaag ggccgagcgc 2040
agaagtggtc ctgcaacttt atccgcctcc atccagtcta ttaattgttg ccgggaagct 2100
agagtaagta gttcgccagt taatagtttg cgcaacgttg ttgccattgc tacaggcatc 2160
gtggtgtcac gctcgtcgtt tggtatggct tcattcagct ccggttccca acgatcaagg 2220
cgagttacat gatcccccat gttgtgcaaa aaagcggtta gctccttcgg tcctccgatc 2280
gttgtcagaa gtaagttggc cgcagtgtta tcactcatgg ttatggcagc actgcataat 2340
tctcttactg tcatgccatc cgtaagatgc ttttctgtga ctggtgagta ctcaaccaag 2400
tcattctgag aatagtgtat gcggcgaccg agttgctctt gcccggcgtc aatacgggat 2460
aataccgcgc cacatagcag aactttaaaa gtgctcatca ttggaaaacg ttcttcgggg 2520

cgaaaactct caaggatctt accgctgttg agatccagtt cgatgtaacc cactcgtgca 2580

cccaactgat cttcagcatc ttttactttc accagcgttt ctgggtgagc aaaaacagga 2640

aggcaaaatg ccgcaaaaaa gggaataagg gcgacacgga aatgttgaat actcatactc 2700

ttcctttttc aatattattg aagcatttat cagggttatt gtctcatgag cggatacata 2760

tttgaatgta tttagaaaaa taaacaaata ggggttccgc gcacatttcc ccgaaaagtg 2820

ccacctgacg tctaagaaac cattattatc atgacattaa cctataaaaa taggcgtatc 2880

acgaggccct ttcgtctcgc gcgtttcggt gatgacggtg aaaacctctg acacatgcag 2940

ctcccggaga cggtcacagc ttgtctgtaa gcggatgccg ggagcagaca agcccgtcag 3000

ggcgcgtcag cgggtgttgg cgggtgtcgg ggctggctta actatgcggc atcagagcag 3060

attgtactga gagtgcacca tatggacata ttgtcgttag aacgcggcta caattaatac 3120

ataaccttat gtatcataca catacgattt aggtgacact ata 3163

<210> 5
<211> 132
<212> DNA
<213> Artificial Sequence

<220>
<223> 5′-AATTC-A120-GATATCA-3′

<400> 5
aattcaaaaa aaaaaaaaaa aaaaaaaaaa aaaaaaaaaa aaaaaaaaaa aaaaaaaaaa 60

aaaaaaaaaa aaaaaaaaaa aaaaaaaaaa aaaaaaaaaa aaaaaaaaaa aaaaaaaaaa 120

aaaaagatat ca 132

<210> 6
<211> 132
<212> DNA
<213> Artificial Sequence

<220>
<223> 5′-GATCTGATATC-T120-G-3′

<400> 6
gatctgatat cttttttttt tttttttttt tttttttttt tttttttttt tttttttttt 60

tttttttttt tttttttttt tttttttttt tttttttttt tttttttttt tttttttttt 120

tttttttttt tg 132

<210> 7
<211> 3162
<212> DNA
<213> Artificial Sequence

<220>
<223> pSP73-GLuc-A(120)-EcoRV

<400> 7
gaaccagatc tgatatcttt tttttttttt tttttttttt tttttttttt tttttttttt 60
tttttttttt tttttttttt tttttttttt tttttttttt tttttttttt tttttttttt 120
tttttttttt ttttttttgaa ttcgagctcg gtacccgggg atcctctaga tgcatgctcg 180
agcggccgct tagtcaccac cggccccctt gatcttgtcc acctggccct ggatcttgct 240
ggcaaaggtc gcacagcgtt gcggcagcca cttcttgagc aggtcagaac actgcacgtt 300
ggcaagccct ttgaggcagc cagttgtgca gtccacacac agatcgacct gtgcgatgaa 360
ctgctccatg ggctccaagt ccttgaaccc aggaatctca ggaatgtcga cgatcgcctc 420
gcctatgccg ccctgtgcgg actctttgtc gccttcgtag gtgtggcagc gtcctgggat 480
gaacttcttc atcttgggcg tgcacttgat gtgggacagg cagatcagac agcccctggt 540
gcagccagct ttccgggcat tggcttccat ctctttgagc acctccagcg gcagcttctt 600
gccgggcaac ttcccgcggt cagcatcgag atccgtggtc gcgaagttgc tggccacggc 660
cacgatgttg aagtcttcgt tgttctcggt gggcttggcc tcggccacag cgatgcagat 720
cagggcaaac agaactttga ctcccatggt ggctggatcc gagctcggta ccaagcttca 780
gctgctcgag gccggtctcc ctatagtgag tcgtattaat ttcgataagc caggttaacc 840
tgcattaatg aatcggccaa cgcgcgggga gaggcggttt gcgtattggg cgctcttccg 900
cttcctcgct cactgactcg ctgcgctcgg tcgttcggct gcggcgagcg gtatcagctc 960
actcaaaggc ggtaatacgg ttatccacag aatcagggga taacgcagga aagaacatgt 1020
gagcaaaagg ccagcaaaag gccaggaacc gtaaaaaggc cgcgttgctg gcgtttttcc 1080
ataggctccg cccccctgac gagcatcaca aaaatcgacg ctcaagtcag aggtggcgaa 1140
acccgacagg actataaaga taccaggcgt ttccccctgg aagctccctc gtgcgctctc 1200
ctgttccgac cctgccgctt accggatacc tgtccgcctt tctcccttcg ggaagcgtgg 1260
cgctttctca tagctcacgc tgtaggtatc tcagttcggt gtaggtcgtt cgctccaagc 1320
tgggctgtgt gcacgaaccc cccgttcagc ccgaccgctg cgccttatcc ggtaactatc 1380
gtcttgagtc caacccggta agacacgact tatcgccact ggcagcagcc actggtaaca 1440
ggattagcag agcgaggtat gtaggcggtg ctacagagtt cttgaagtgg tggcctaact 1500
acggctacac tagaagaaca gtatttggta tctgcgctct gctgaagcca gttaccttcg 1560
gaaaaagagt tggtagctct tgatccggca aacaaaccac cgctggtagc ggtggttttt 1620
ttgtttgcaa gcagcagatt acgcgcagaa aaaaaggatc tcaagaagat cctttgatct 1680
tttctacggg gtctgacgct cagtggaacg aaaactcacg ttaagggatt ttggtcatga 1740
gattatcaaa aggatcttc acctagatcc ttttaaatta aaaatgaagt tttaaatcaa 1800
tctaaagtat atatgagtaa acttggtctg acagttacca atgcttaatc agtgaggcac 1860

ctatctcagc gatctgtcta tttcgttcat ccatagttgc ctgactcccc gtcgtgtaga 1920

taactacgat acgggagggc ttaccatctg gccccagtgc tgcaatgata ccgcgagacc 1980

cacgctcacc ggctccagat ttatcagcaa taaaccagcc agccggaagg gccgagcgca 2040

gaagtggtcc tgcaacttta tccgcctcca tccagtctat taattgttgc cgggaagcta 2100

gagtaagtag ttcgccagtt aatagtttgc gcaacgttgt tgccattgct acaggcatcg 2160

tggtgtcacg ctcgtcgttt ggtatggctt cattcagctc cggttcccaa cgatcaaggc 2220

gagttacatg atcccccatg ttgtgcaaaa aagcggttag ctccttcggt cctccgatcg 2280

ttgtcagaag taagttggcc gcagtgttat cactcatggt tatggcagca ctgcataatt 2340

ctcttactgt catgccatcc gtaagatgct tttctgtgac tggtgagtac tcaaccaagt 2400

cattctgaga atagtgtatg cggcgaccga gttgctcttg cccggcgtca atacgggata 2460

ataccgcgcc acatagcaga actttaaaag tgctcatcat tggaaaacgt tcttcggggc 2520

gaaaactctc aaggatctta ccgctgttga gatccagttc gatgtaaccc actcgtgcac 2580

ccaactgatc ttcagcatct tttactttca ccagcgtttc tgggtgagca aaaacaggaa 2640

ggcaaaatgc cgcaaaaaag ggaataaggg cgacacggaa atgttgaata ctcatactct 2700

tcctttttca atattattga agcatttatc agggttattg tctcatgagc ggatacatat 2760

ttgaatgtat ttagaaaaat aaacaaatag gggttccgcg cacatttccc cgaaaagtgc 2820

cacctgacgt ctaagaaacc attattatca tgacattaac ctataaaaat aggcgtatca 2880

cgaggccctt tcgtctcgcg cgtttcggtg atgacggtga aaacctctga cacatgcagc 2940

tcccggagac ggtcacagct tgtctgtaag cggatgccgg gagcagacaa gcccgtcagg 3000

gcgcgtcagc gggtgttggc gggtgtcggg gctggcttaa ctatgcggca tcagagcaga 3060

ttgtactgag agtgcaccat atggacatat tgtcgttaga acgcggctac aattaataca 3120

taaccttatg tatcatacac atacgattta ggtgacacta ta 3162

<210> 8
<211> 69
<212> DNA
<213> Artificial Sequence

<220>
<223> 5′-G-A61-GAGACGA-3′

<400> 8
gaaaaaaaaa aaaaaaaaaa aaaaaaaaaa aaaaaaaaaa aaaaaaaaaa aaaaaaaaaa 60

aagagacga 69

<210> 9
<211> 73
<212> DNA

<213> Artificial Sequence

<220>
<223> 5'-GATCTCGTCTC-T61-C-3

<400> 9
gatctcgtct cttttttttt tttttttttt tttttttttt tttttttttt tttttttttt 60

tttttttttt ttc 73

<210> 10
<211> 3227
<212> DNA
<213> Artificial Sequence

<220>
<223> pSP73-GLuc-A(120)-EcoRV+T61

<400> 10
gaaccagatc tcgtctcttt tttttttttt tttttttttt tttttttttt tttttttttt 60

tttttttttt tttttttca tctttttttt tttttttttt tttttttttt tttttttttt 120

tttttttttt tttttttttt tttttttttt tttttttttt tttttttttt tttttttttt 180

tttttttttt tttttttttt ttgaattcga gctcggtacc cggggatcct ctagatgcat 240

gctcgagcgg ccgcttagtc accaccggcc cccttgatct tgtccacctg gccctggatc 300

ttgctggcaa aggtcgcaca gcgttgcggc agccacttct tgagcaggtc agaacactgc 360

acgttggcaa gccctttgag gcagccagtt gtgcagtcca cacacagatc gacctgtgcg 420

atgaactgct ccatgggctc caagtccttg aacccaggaa tctcaggaat gtcgacgatc 480

gcctcgccta tgccgccctg tgcggactct ttgtcgcctt cgtaggtgtg gcagcgtcct 540

gggatgaact tcttcatctt gggcgtgcac ttgatgtggg acaggcagat cagacagccc 600

ctggtgcagc cagctttccg ggcattggct tccatctctt tgagcacctc cagcggcagc 660

ttcttgccgg gcaacttccc gcggtcagca tcgagatccg tggtcgcgaa gttgctggcc 720

acggccacga tgttgaagtc ttcgttgttc tcggtgggct tggcctcggc cacagcgatg 780

cagatcaggg caaacagaac tttgactccc atggtggctg gatccgagct cggtaccaag 840

cttcagctgc tcgaggccgg tctccctata gtgagtcgta ttaatttcga taagccaggt 900

taacctgcat taatgaatcg gccaacgcgc ggggagaggc ggtttgcgta ttgggcgctc 960

ttccgcttcc tcgctcactg actcgctgcg ctcggtcgtt cggctgcggc gagcggtatc 1020

agctcactca aaggcggtaa tacggttatc cacagaatca ggggataacg caggaaagaa 1080

catgtgagca aaaggccagc aaaaggccag gaaccgtaaa aaggccgcgt tgctggcgtt 1140

tttccatagg ctccgccccc ctgacgagca tcacaaaaat cgacgctcaa gtcagaggtg 1200

gcgaaacccg acaggactat aaagatacca ggcgtttccc cctggaagct ccctcgtgcg 1260

ctctcctgtt ccgaccctgc cgcttaccgg atacctgtcc gcctttctcc cttcgggaag 1320

cgtggcgctt tctcatagct cacgctgtag gtatctcagt tcggtgtagg tcgttcgctc 1380
caagctgggc tgtgtgcacg aaccccccgt tcagcccgac cgctgcgcct tatccggtaa 1440
ctatcgtctt gagtccaacc cggtaagaca cgacttatcg ccactggcag cagccactgg 1500
taacaggatt agcagagcga ggtatgtagg cggtgctaca gagttcttga agtggtggcc 1560
taactacggc tacactagaa gaacagtatt tggtatctgc gctctgctga agccagttac 1620
cttcggaaaa agagttggta gctcttgatc cggcaaacaa accaccgctg gtagcggtgg 1680
tttttttgtt tgcaagcagc agattacgcg cagaaaaaaa ggatctcaag aagatccttt 1740
gatcttttct acggggtctg acgctcagtg gaacgaaaac tcacgttaag ggattttggt 1800
catgagatta tcaaaaagga tcttcaccta gatcctttta aattaaaaat gaagttttaa 1860
atcaatctaa agtatatatg agtaaacttg gtctgacagt taccaatgct taatcagtga 1920
ggcacctatc tcagcgatct gtctatttcg ttcatccata gttgcctgac tccccgtcgt 1980
gtagataact acgatacggg agggcttacc atctggcccc agtgctgcaa tgataccgcg 2040
agacccacgc tcaccggctc cagatttatc agcaataaac cagccagccg gaagggccga 2100
gcgcagaagt ggtcctgcaa ctttatccgc ctccatccag tctattaatt gttgccggga 2160
agctagagta agtagttcgc cagttaatag tttgcgcaac gttgttgcca ttgctacagg 2220
catcgtggtg tcacgctcgt cgtttggtat ggcttcattc agctccggtt cccaacgatc 2280
aaggcgagtt acatgatccc ccatgttgtg caaaaaagcg gttagctcct tcggtcctcc 2340
gatcgttgtc agaagtaagt tggccgcagt gttatcactc atggttatgg cagcactgca 2400
taattctctt actgtcatgc catccgtaag atgcttttct gtgactggtg agtactcaac 2460
caagtcattc tgagaatagt gtatgcggcg accgagttgc tcttgcccgg cgtcaatacg 2520
ggataatacc gcgccacata gcagaacttt aaaagtgctc atcattggaa aacgttcttc 2580
ggggcgaaaa ctctcaagga tcttaccgct gttgagatcc agttcgatgt aacccactcg 2640
tgcacccaac tgatcttcag catcttttac tttcaccagc gtttctgggt gagcaaaaac 2700
aggaaggcaa aatgccgcaa aaaagggaat aagggcgaca cggaaatgtt gaatactcat 2760
actcttcctt tttcaatatt attgaagcat ttatcagggt tattgtctca tgagcggata 2820
catatttgaa tgtatttaga aaaataaaca aataggggtt ccgcgcacat ttccccgaaa 2880
agtgccacct gacgtctaag aaaccattat tatcatgaca ttaacctata aaaataggcg 2940
tatcacgagg ccctttcgtc tcgcgcgttt cggtgatgac ggtgaaaacc tctgacacat 3000
gcagctcccg gagacggtca cagcttgtct gtaagcggat gccgggagca gacaagcccg 3060
tcagggcgcg tcagcgggtg ttggcgggtg tcggggctgg cttaactatg cggcatcaga 3120
gcagattgta ctgagagtgc accatatgga catattgtcg ttagaacgcg gctacaatta 3180

atacataacc ttatgtatca tacacatacg atttaggtga cactata 3227

<210> 11
<211> 99
<212> DNA
<213> Artificial Sequence

<220>
<223> 5'-G-A91-GAGACGA-3'

<400> 11
gaaaaaaaaa aaaaaaaaaa aaaaaaaaaa aaaaaaaaaa aaaaaaaaaa aaaaaaaaaa 60

aaaaaaaaaa aaaaaaaaaa aaaaaaaaaa aagagacga 99

<210> 12
<211> 103
<212> DNA
<213> Artificial Sequence

<220>
<223> 5'-GATCTCGTCTC-T91-C-3

<400> 12
gatctcgtct cttttttttt tttttttttt tttttttttt tttttttttt tttttttttt 60

tttttttttt tttttttttt tttttttttt tttttttttt ttc 103

<210> 13
<211> 3257
<212> DNA
<213> Artificial Sequence

<220>
<223> pSP73-GLuc-A(120)-EcoRV+T91

<400> 13
gaaccagatc tcgtctcttt tttttttttt tttttttttt tttttttttt tttttttttt 60

tttttttttt tttttttttt tttttttttt tttttttttt tttttttttca tctttttttt 120

tttttttttt tttttttttt tttttttttt tttttttttt tttttttttt tttttttttt 180

tttttttttt tttttttttt tttttttttt tttttttttt tttttttttt ttgaattcga 240

gctcggtacc cggggatcct ctagatgcat gctcgagcgg ccgcttagtc accaccggcc 300

cccttgatct tgtccacctg gccctggatc ttgctggcaa aggtcgcaca gcgttgcggc 360

agccacttct tgagcaggtc agaacactgc acgttggcaa gccctttgag gcagccagtt 420

gtgcagtcca cacacagatc gacctgtgcg atgaactgct ccatgggctc caagtccttg 480

aacccaggaa tctcaggaat gtcgacgatc gcctcgccta tgccgccctg tgcggactct 540

ttgtcgcctt cgtaggtgtg gcagcgtcct gggatgaact tcttcatctt gggcgtgcac 600

ttgatgtggg acaggcagat cagacagccc ctggtgcagc cagctttccg ggcattggct 660

tccatctctt tgagcacctc cagcggcagc ttcttgccgg gcaacttccc gcggtcagca 720

```
tcgagatccg tggtcgcgaa gttgctggcc acggccacga tgttgaagtc ttcgttgttc      780
tcggtgggct tggcctcggc cacagcgatg cagatcaggg caaacagaac tttgactccc      840
atggtggctg gatccgagct cggtaccaag cttcagctgc tcgaggccgg tctccctata      900
gtgagtcgta ttaatttcga taagccaggt taacctgcat taatgaatcg gccaacgcgc      960
ggggagaggc ggtttgcgta ttgggcgctc ttccgcttcc tcgctcactg actcgctgcg     1020
ctcggtcgtt cggctgcggc gagcggtatc agctcactca aaggcggtaa tacggttatc     1080
cacagaatca ggggataacg caggaaagaa catgtgagca aaaggccagc aaaaggccag     1140
gaaccgtaaa aaggccgcgt tgctggcgtt tttccatagg ctccgccccc ctgacgagca     1200
tcacaaaaat cgacgctcaa gtcagaggtg gcgaaacccg acaggactat aaagatacca     1260
ggcgtttccc cctggaagct ccctcgtgcg ctctcctgtt ccgaccctgc cgcttaccgg     1320
atacctgtcc gcctttctcc cttcgggaag cgtggcgctt tctcatagct cacgctgtag     1380
gtatctcagt tcggtgtagg tcgttcgctc caagctgggc tgtgtgcacg aaccccccgt     1440
tcagcccgac cgctgcgcct tatccggtaa ctatcgtctt gagtccaacc cggtaagaca     1500
cgacttatcg ccactggcag cagccactgg taacaggatt agcagagcga ggtatgtagg     1560
cggtgctaca gagttcttga agtggtggcc taactacggc tacactagaa gaacagtatt     1620
tggtatctgc gctctgctga agccagttac cttcggaaaa agagttggta gctcttgatc     1680
cggcaaacaa accaccgctg gtagcggtgg tttttttgtt tgcaagcagc agattacgcg     1740
cagaaaaaaa ggatctcaag aagatccttt gatcttttct acggggtctg acgctcagtg     1800
gaacgaaaac tcacgttaag ggattttggt catgagatta tcaaaaagga tcttcaccta     1860
gatcctttta aattaaaaat gaagttttaa atcaatctaa agtatatatg agtaaacttg     1920
gtctgacagt taccaatgct taatcagtga ggcacctatc tcagcgatct gtctatttcg     1980
ttcatccata gttgcctgac tccccgtcgt gtagataact acgatacggg agggcttacc     2040
atctggcccc agtgctgcaa tgataccgcg agacccacgc tcaccggctc cagatttatc     2100
agcaataaac cagccagccg gaagggccga gcgcagaagt ggtcctgcaa ctttatccgc     2160
ctccatccag tctattaatt gttgccggga agctagagta agtagttcgc cagttaatag     2220
tttgcgcaac gttgttgcca ttgctacagg catcgtggtg tcacgctcgt cgtttggtat     2280
ggcttcattc agctccggtt cccaacgatc aaggcgagtt acatgatccc ccatgttgtg     2340
caaaaaagcg gttagctcct tcggtcctcc gatcgttgtc agaagtaagt tggccgcagt     2400
gttatcactc atggttatgg cagcactgca taattctctt actgtcatgc catccgtaag     2460
atgcttttct gtgactggtg agtactcaac caagtcattc tgagaatagt gtatgcggcg     2520
accgagttgc tcttgcccgg cgtcaatacg ggataatacc gcgccacata gcagaacttt     2580
```

aaaagtgctc atcattggaa aacgttcttc ggggcgaaaa ctctcaagga tcttaccgct 2640

gttgagatcc agttcgatgt aacccactcg tgcacccaac tgatcttcag catcttttac 2700

tttcaccagc gtttctgggt gagcaaaaac aggaaggcaa aatgccgcaa aaaagggaat 2760

aagggcgaca cggaaatgtt gaatactcat actcttcctt tttcaatatt attgaagcat 2820

ttatcagggt tattgtctca tgagcggata catatttgaa tgtatttaga aaaataaaca 2880

aataggggtt ccgcgcacat ttccccgaaa agtgccacct gacgtctaag aaaccattat 2940

tatcatgaca ttaacctata aaaataggcg tatcacgagg ccctttcgtc tcgcgcgttt 3000

cggtgatgac ggtgaaaacc tctgacacat gcagctcccg gagacggtca cagcttgtct 3060

gtaagcggat gccgggagca gacaagcccg tcagggcgcg tcagcgggtg ttggcgggtg 3120

tcggggctgg cttaactatg cggcatcaga gcagattgta ctgagagtgc accatatgga 3180

catattgtcg ttagaacgcg gctacaatta atacataacc ttatgtatca tacacatacg 3240

atttaggtga cactata 3257

<210> 14
<211> 129
<212> DNA
<213> Artificial Sequence

<220>
<223> 5′-G-A121-GAGACGA-3′

<400> 14
gaaaaaaaaa aaaaaaaaaa aaaaaaaaaa aaaaaaaaaa aaaaaaaaaa aaaaaaaaaa 60

aaaaaaaaaa aaaaaaaaaa aaaaaaaaaa aaaaaaaaaa aaaaaaaaaa aaaaaaaaaa 120

aagagacga 129

<210> 15
<211> 133
<212> DNA
<213> Artificial Sequence

<220>
<223> 5′-GATCTCGTCTC-T121-C-3

<400> 15
gatctcgtct cttttttttt tttttttttt tttttttttt tttttttttt tttttttttt 60

tttttttttt tttttttttt tttttttttt tttttttttt tttttttttt tttttttttt 120

tttttttttt ttc 133

<210> 16
<211> 3287
<212> DNA
<213> Artificial Sequence

<220>

<223> pSP73-GLuc-A(120)-EcoRV+T121

<400> 16

```
gaaccagatc tcgtctcttt tttttttttt tttttttttt tttttttttt tttttttttt      60
tttttttttt tttttttttt tttttttttt tttttttttt tttttttttt tttttttttt     120
tttttttttt ttttttttca tctttttttt tttttttttt tttttttttt tttttttttt     180
tttttttttt tttttttttt tttttttttt tttttttttt tttttttttt tttttttttt     240
tttttttttt tttttttttt ttgaattcga gctcggtacc cggggatcct ctagatgcat     300
gctcgagcgg ccgcttagtc accaccggcc cccttgatct tgtccacctg gccctggatc     360
ttgctggcaa aggtcgcaca gcgttgcggc agccacttct tgagcaggtc agaacactgc     420
acgttggcaa gccctttgag gcagccagtt gtgcagtcca cacacagatc gacctgtgcg     480
atgaactgct ccatgggctc caagtccttg aacccaggaa tctcaggaat gtcgacgatc     540
gcctcgccta tgccgccctg tgcggactct ttgtcgcctt cgtaggtgtg gcagcgtcct     600
gggatgaact tcttcatctt gggcgtgcac ttgatgtggg acaggcagat cagacagccc     660
ctggtgcagc cagctttccg ggcattggct tccatctctt tgagcacctc cagcggcagc     720
ttcttgccgg gcaacttccc gcggtcagca tcgagatccg tggtcgcgaa gttgctggcc     780
acggccacga tgttgaagtc ttcgttgttc tcggtgggct tggcctcggc cacagcgatg     840
cagatcaggg caaacagaac tttgactccc atggtggctg gatccgagct cggtaccaag     900
cttcagctgc tcgaggccgg tctccctata gtgagtcgta ttaatttcga taagccaggt     960
taacctgcat taatgaatcg gccaacgcgc ggggagaggc ggtttgcgta ttgggcgctc    1020
ttccgcttcc tcgctcactg actcgctgcg ctcggtcgtt cggctgcggc gagcggtatc    1080
agctcactca aaggcggtaa tacggttatc cacagaatca ggggataacg caggaaagaa    1140
catgtgagca aaaggccagc aaaaggccag gaaccgtaaa aaggccgcgt tgctggcgtt    1200
tttccatagg ctccgccccc ctgacgagca tcacaaaaat cgacgctcaa gtcagaggtg    1260
gcgaaacccg acaggactat aaagatacca ggcgtttccc cctggaagct ccctcgtgcg    1320
ctctcctgtt ccgaccctgc cgcttaccgg atacctgtcc gcctttctcc cttcgggaag    1380
cgtggcgctt tctcatagct cacgctgtag gtatctcagt tcggtgtagg tcgttcgctc    1440
caagctgggc tgtgtgcacg aaccccccgt tcagcccgac cgctgcgcct tatccggtaa    1500
ctatcgtctt gagtccaacc cggtaagaca cgacttatcg ccactggcag cagccactgg    1560
taacaggatt agcagagcga ggtatgtagg cggtgctaca gagttcttga agtggtggcc    1620
taactacggc tacactagaa gaacagtatt tggtatctgc gctctgctga agccagttac    1680
cttcggaaaa agagttggta gctcttgatc cggcaaacaa accaccgctg gtagcggtgg    1740
tttttttgtt tgcaagcagc agattacgcg cagaaaaaaa ggatctcaag aagatccttt    1800
```

gatcttttct acggggtctg acgctcagtg gaacgaaaac tcacgttaag ggattttggt 1860

catgagatta tcaaaaagga tcttcaccta gatcctttta aattaaaaat gaagttttaa 1920

atcaatctaa agtatatatg agtaaacttg gtctgacagt taccaatgct taatcagtga 1980

ggcacctatc tcagcgatct gtctatttcg ttcatccata gttgcctgac tccccgtcgt 2040

gtagataact acgatacggg agggcttacc atctggcccc agtgctgcaa tgataccgcg 2100

agacccacgc tcaccggctc cagatttatc agcaataaac cagccagccg gaagggccga 2160

gcgcagaagt ggtcctgcaa ctttatccgc ctccatccag tctattaatt gttgccggga 2220

agctagagta agtagttcgc cagttaatag tttgcgcaac gttgttgcca ttgctacagg 2280

catcgtggtg tcacgctcgt cgtttggtat ggcttcattc agctccggtt cccaacgatc 2340

aaggcgagtt acatgatccc ccatgttgtg caaaaaagcg gttagctcct tcggtcctcc 2400

gatcgttgtc agaagtaagt tggccgcagt gttatcactc atggttatgg cagcactgca 2460

taattctctt actgtcatgc catccgtaag atgcttttct gtgactggtg agtactcaac 2520

caagtcattc tgagaatagt gtatgcggcg accgagttgc tcttgcccgg cgtcaatacg 2580

ggataatacc gcgccacata gcagaacttt aaaagtgctc atcattggaa aacgttcttc 2640

ggggcgaaaa ctctcaagga tcttaccgct gttgagatcc agttcgatgt aacccactcg 2700

tgcacccaac tgatcttcag catcttttac tttcaccagc gtttctgggt gagcaaaaac 2760

aggaaggcaa aatgccgcaa aaaagggaat aagggcgaca cggaaatgtt gaatactcat 2820

actcttcctt tttcaatatt attgaagcat ttatcagggt tattgtctca tgagcggata 2880

catatttgaa tgtatttaga aaaataaaca aataggggtt ccgcgcacat ttccccgaaa 2940

agtgccacct gacgtctaag aaaccattat tatcatgaca ttaacctata aaaataggcg 3000

tatcacgagg ccctttcgtc tcgcgcgttt cggtgatgac ggtgaaaacc tctgacacat 3060

gcagctcccg gagacggtca cagcttgtct gtaagcggat gccgggagca gacaagcccg 3120

tcagggcgcg tcagcgggtg ttggcgggtg tcggggctgg cttaactatg cggcatcaga 3180

gcagattgta ctgagagtgc accatatgga catattgtcg ttagaacgcg gctacaatta 3240

atacataacc ttatgtatca tacacatacg atttaggtga cactata 3287

<210> 17
<211> 128
<212> DNA
<213> Artificial Sequence

<220>
<223> 5'-G-A120-GATATCA-3'

<400> 17
gaaaaaaaaa aaaaaaaaaa aaaaaaaaaa aaaaaaaaaa aaaaaaaaaa aaaaaaaaaa 60

aaaaaaaaaa aaaaaaaaaa aaaaaaaaaa aaaaaaaaaa aaaaaaaaaa aaaaaaaaaa 120

agatatca 128

<210> 18
<211> 132
<212> DNA
<213> Artificial Sequence

<220>
<223> 5′-GATCTGATATC-T120-C-3′

<400> 18
gatctgatat cttttttttt tttttttttt tttttttttt tttttttttt tttttttttt 60

tttttttttt tttttttttt tttttttttt tttttttttt tttttttttt tttttttttt 120

tttttttttt tc 132

<210> 19
<211> 3286
<212> DNA
<213> Artificial Sequence

<220>
<223> pSP73-GLuc-A(240)-EcoRV

<400> 19
gaaccagatc tgatatcttt tttttttttt tttttttttt tttttttttt tttttttttt 60

tttttttttt tttttttttt tttttttttt tttttttttt tttttttttt tttttttttt 120

tttttttttt tttttttcat cttttttttt tttttttttt tttttttttt tttttttttt 180

tttttttttt tttttttttt tttttttttt tttttttttt tttttttttt tttttttttt 240

tttttttttt tttttttttt tgaattcgag ctcggtaccc ggggatcctc tagatgcatg 300

ctcgagcggc cgcttagtca ccaccggccc ccttgatctt gtccacctgg ccctggatct 360

tgctggcaaa ggtcgcacag cgttgcggca gccacttctt gagcaggtca gaacactgca 420

cgttggcaag ccctttgagg cagccagttg tgcagtccac acacagatcg acctgtgcga 480

tgaactgctc catgggctcc aagtccttga acccaggaat ctcaggaatg tcgacgatcg 540

cctcgcctat gccgccctgt gcggactctt tgtcgccttc gtaggtgtgg cagcgtcctg 600

ggatgaactt cttcatcttg ggcgtgcact tgatgtggga caggcagatc agacagcccc 660

tggtgcagcc agctttccgg gcattggctt ccatctcttt gagcacctcc agcggcagct 720

tcttgccggg caacttcccg cggtcagcat cgagatccgt ggtcgcgaag ttgctggcca 780

cggccacgat gttgaagtct tcgttgttct cggtgggctt ggcctcggcc acagcgatgc 840

agatcagggc aaacagaact ttgactccca tggtggctgg atccgagctc ggtaccaagc 900

ttcagctgct cgaggccggt ctccctatag tgagtcgtat taatttcgat aagccaggtt 960

aacctgcatt aatgaatcgg ccaacgcgcg gggagaggcg gtttgcgtat tgggcgctct 1020

tccgcttcct cgctcactga ctcgctgcgc tcggtcgttc ggctgcggcg agcggtatca 1080
gctcactcaa aggcggtaat acggttatcc acagaatcag gggataacgc aggaaagaac 1140
atgtgagcaa aaggccagca aaaggccagg aaccgtaaaa aggccgcgtt gctggcgttt 1200
ttccataggc tccgcccccc tgacgagcat cacaaaaatc gacgctcaag tcagaggtgg 1260
cgaaacccga caggactata aagataccag gcgtttcccc ctggaagctc cctcgtgcgc 1320
tctcctgttc cgaccctgcc gcttaccgga tacctgtccg cctttctccc ttcgggaagc 1380
gtggcgcttt ctcatagctc acgctgtagg tatctcagtt cggtgtaggt cgttcgctcc 1440
aagctgggct gtgtgcacga accccccgtt cagcccgacc gctgcgcctt atccggtaac 1500
tatcgtcttg agtccaaccc ggtaagacac gacttatcgc cactggcagc agccactggt 1560
aacaggatta gcagagcgag gtatgtaggc ggtgctacag agttcttgaa gtggtggcct 1620
aactacggct acactagaag aacagtattt ggtatctgcg ctctgctgaa gccagttacc 1680
ttcggaaaaa gagttggtag ctcttgatcc ggcaaacaaa ccaccgctgg tagcggtggt 1740
tttttgttt gcaagcagca gattacgcgc agaaaaaaag gatctcaaga agatcctttg 1800
atcttttcta cggggtctga cgctcagtgg aacgaaaact cacgttaagg gattttggtc 1860
atgagattat caaaaaggat cttcacctag atccttttaa attaaaaatg aagttttaaa 1920
tcaatctaaa gtatatatga gtaaacttgg tctgacagtt accaatgctt aatcagtgag 1980
gcacctatct cagcgatctg tctatttcgt tcatccatag ttgcctgact ccccgtcgtg 2040
tagataacta cgatacggga gggcttacca tctggcccca gtgctgcaat gataccgcga 2100
gacccacgct caccggctcc agatttatca gcaataaacc agccagccgg aagggccgag 2160
cgcagaagtg gtcctgcaac tttatccgcc tccatccagt ctattaattg ttgccgggaa 2220
gctagagtaa gtagttcgcc agttaatagt ttgcgcaacg ttgttgccat tgctacaggc 2280
atcgtggtgt cacgctcgtc gtttggtatg gcttcattca gctccggttc ccaacgatca 2340
aggcgagtta catgatcccc catgttgtgc aaaaaagcgg ttagctcctt cggtcctccg 2400
atcgttgtca gaagtaagtt ggccgcagtg ttatcactca tggttatggc agcactgcat 2460
aattctctta ctgtcatgcc atccgtaaga tgcttttctg tgactggtga gtactcaacc 2520
aagtcattct gagaatagtg tatgcggcga ccgagttgct cttgcccggc gtcaatacgg 2580
gataataccg cgccacatag cagaacttta aaagtgctca tcattggaaa acgttcttcg 2640
gggcgaaaac tctcaaggat cttaccgctg ttgagatcca gttcgatgta acccactcgt 2700
gcacccaact gatcttcagc atcttttact ttcaccagcg tttctgggtg agcaaaaaca 2760
ggaaggcaaa atgccgcaaa aaagggaata agggcgacac ggaaatgttg aatactcata 2820
ctcttccttt ttcaatatta ttgaagcatt tatcagggtt attgtctcat gagcggatac 2880
atatttgaat gtatttagaa aaataaacaa ataggggttc cgcgcacatt tccccgaaaa 2940

gtgccacctg acgtctaaga aaccattatt atcatgacat taacctataa aaataggcgt 3000

atcacgaggc cctttcgtct cgcgcgtttc ggtgatgacg gtgaaaacct ctgacacatg 3060

cagctcccgg agacggtcac agcttgtctg taagcggatg ccgggagcag acaagcccgt 3120

cagggcgcgt cagcgggtgt tggcgggtgt cggggctggc ttaactatgc ggcatcagag 3180

cagattgtac tgagagtgca ccatatggac atattgtcgt tagaacgcgg ctacaattaa 3240

tacataacct tatgtatcat acacatacga tttaggtgac actata 3286

<210> 20
<211> 39
<212> DNA
<213> Artificial Sequence

<220>
<223> 5′-G-A31-GAGACGA-3′

<400> 20
gaaaaaaaaa aaaaaaaaaa aaaaaaaaaa aagagacga 39

<210> 21
<211> 43
<212> DNA
<213> Artificial Sequence

<220>
<223> 5′-GATCTCGTCTC-T31-C-3′

<400> 21
gatctcgtct cttttttttt tttttttttt tttttttttt ttc 43

<210> 22
<211> 3321
<212> DNA
<213> Artificial Sequence

<220>
<223> pSP73-GLuc-A(240)-EcoRV+T31

<400> 22
gaaccagatc tcgtctcttt tttttttttt tttttttttt ttttttttca tctttttttt 60

tttttttttt tttttttttt tttttttttt tttttttttt tttttttttt tttttttttt 120

tttttttttt tttttttttt tttttttttt tttttttttt tttttttttt ttcatctttt 180

tttttttttt tttttttttt tttttttttt tttttttttt tttttttttt tttttttttt 240

tttttttttt tttttttttt tttttttttt tttttttttt tttttttttt ttttttgaat 300

tcgagctcgg tacccgggga tcctctagat gcatgctcga gcggccgctt agtcaccacc 360

ggcccccttg atcttgtcca cctggccctg gatcttgctg gcaaaggtcg cacagcgttg 420

cggcagccac ttcttgagca ggtcagaaca ctgcacgttg gcaagccctt tgaggcagcc 480

agttgtgcag tccacacaca gatcgacctg tgcgatgaac tgctccatgg gctccaagtc 540
cttgaaccca ggaatctcag gaatgtcgac gatcgcctcg cctatgccgc cctgtgcgga 600
ctctttgtcg ccttcgtagg tgtggcagcg tcctgggatg aacttcttca tcttgggcgt 660
gcacttgatg tgggacaggc agatcagaca gcccctggtg cagccagctt tccgggcatt 720
ggcttccatc tctttgagca cctccagcgg cagcttcttg ccgggcaact tcccgcggtc 780
agcatcgaga tccgtggtcg cgaagttgct ggccacggcc acgatgttga agtcttcgtt 840
gttctcggtg ggcttggcct cggccacagc gatgcagatc agggcaaaca gaactttgac 900
tcccatggtg gctggatccg agctcggtac caagcttcag ctgctcgagg ccggtctccc 960
tatagtgagt cgtattaatt tcgataagcc aggttaacct gcattaatga atcggccaac 1020
gcgcggggag aggcggtttg cgtattgggc gctcttccgc ttcctcgctc actgactcgc 1080
tgcgctcggt cgttcggctg cggcgagcgg tatcagctca ctcaaaggcg gtaatacggt 1140
tatccacaga atcaggggat aacgcaggaa agaacatgtg agcaaaaggc cagcaaaagg 1200
ccaggaaccg taaaaaggcc gcgttgctgg cgtttttcca taggctccgc ccccctgacg 1260
agcatcacaa aaatcgacgc tcaagtcaga ggtggcgaaa cccgacagga ctataaagat 1320
accaggcgtt tccccctgga agctccctcg tgcgctctcc tgttccgacc ctgccgctta 1380
ccggatacct gtccgccttt ctcccttcgg gaagcgtggc gctttctcat agctcacgct 1440
gtaggtatct cagttcggtg taggtcgttc gctccaagct gggctgtgtg cacgaacccc 1500
ccgttcagcc cgaccgctgc gccttatccg gtaactatcg tcttgagtcc aacccggtaa 1560
gacacgactt atcgccactg gcagcagcca ctggtaacag gattagcaga gcgaggtatg 1620
taggcggtgc tacagagttc ttgaagtggt ggcctaacta cggctacact agaagaacag 1680
tatttggtat ctgcgctctg ctgaagccag ttaccttcgg aaaaagagtt ggtagctctt 1740
gatccggcaa acaaaccacc gctggtagcg gtggtttttt tgtttgcaag cagcagatta 1800
cgcgcagaaa aaaaggatct caagaagatc ctttgatctt ttctacgggg tctgacgctc 1860
agtggaacga aaactcacgt taagggattt tggtcatgag attatcaaaa aggatcttca 1920
cctagatcct tttaaattaa aaatgaagtt ttaaatcaat ctaaagtata tatgagtaaa 1980
cttggtctga cagttaccaa tgcttaatca gtgaggcacc tatctcagcg atctgtctat 2040
ttcgttcatc catagttgcc tgactccccg tcgtgtagat aactacgata cgggagggct 2100
taccatctgg ccccagtgct gcaatgatac cgcgagaccc acgctcaccg gctccagatt 2160
tatcagcaat aaaccagcca gccggaaggg ccgagcgcag aagtggtcct gcaactttat 2220
ccgcctccat ccagtctatt aattgttgcc gggaagctag agtaagtagt tcgccagtta 2280
atagtttgcg caacgttgtt gccattgcta caggcatcgt ggtgtcacgc tcgtcgtttg 2340
gtatggcttc attcagctcc ggttcccaac gatcaaggcg agttacatga tcccccatgt 2400

tgtgcaaaaa agcggttagc tccttcggtc ctccgatcgt tgtcagaagt aagttggccg 2460

cagtgttatc actcatggtt atggcagcac tgcataattc tcttactgtc atgccatccg 2520

taagatgctt ttctgtgact ggtgagtact caaccaagtc attctgagaa tagtgtatgc 2580

ggcgaccgag ttgctcttgc ccggcgtcaa tacgggataa taccgcgcca catagcagaa 2640

ctttaaaagt gctcatcatt ggaaaacgtt cttcggggcg aaaactctca aggatcttac 2700

cgctgttgag atccagttcg atgtaaccca ctcgtgcacc caactgatct tcagcatctt 2760

ttactttcac cagcgtttct gggtgagcaa aaacaggaag gcaaaatgcc gcaaaaaagg 2820

gaataagggc gacacggaaa tgttgaatac tcatactctt cctttttcaa tattattgaa 2880

gcatttatca gggttattgt ctcatgagcg gatacatatt tgaatgtatt tagaaaaata 2940

aacaaatagg ggttccgcgc acatttcccc gaaaagtgcc acctgacgtc taagaaacca 3000

ttattatcat gacattaacc tataaaaata ggcgtatcac gaggcccttt cgtctcgcgc 3060

gtttcggtga tgacggtgaa aacctctgac acatgcagct cccggagacg gtcacagctt 3120

gtctgtaagc ggatgccggg agcagacaag cccgtcaggg cgcgtcagcg ggtgttggcg 3180

ggtgtcgggg ctggcttaac tatgcggcat cagagcagat tgtactgaga gtgcaccata 3240

tggacatatt gtcgttagaa cgcggctaca attaatacat aaccttatgt atcatacaca 3300

tacgatttag gtgacactat a 3321

<210> 23
<211> 3411
<212> DNA
<213> Artificial Sequence

<220>
<223> pSP73-GLuc-A(240)-EcoRV+T121

<400> 23
gaaccagatc tcgtctcttt tttttttttt tttttttttt tttttttttt tttttttttt 60

tttttttttt tttttttttt tttttttttt tttttttttt tttttttttt tttttttttt 120

tttttttttt ttttttttca tctttttttt tttttttttt tttttttttt tttttttttt 180

tttttttttt tttttttttt tttttttttt tttttttttt tttttttttt tttttttttt 240

tttttttttt tttttttttt ttcatctttt tttttttttt tttttttttt tttttttttt 300

tttttttttt tttttttttt tttttttttt tttttttttt tttttttttt tttttttttt 360

tttttttttt tttttttttt ttttttgaat tcgagctcgg tacccgggga tcctctagat 420

gcatgctcga gcggccgctt agtcaccacc ggcccccttg atcttgtcca cctggccctg 480

gatcttgctg gcaaaggtcg cacagcgttg cggcagccac ttcttgagca ggtcagaaca 540

ctgcacgttg gcaagccctt tgaggcagcc agttgtgcag tccacacaca gatcgacctg 600

tgcgatgaac tgctccatgg gctccaagtc cttgaaccca ggaatctcag gaatgtcgac 660
gatcgcctcg cctatgccgc cctgtgcgga ctctttgtcg ccttcgtagg tgtggcagcg 720
tcctgggatg aacttcttca tcttgggcgt gcacttgatg tgggacaggc agatcagaca 780
gcccctggtg cagccagctt tccgggcatt ggcttccatc tctttgagca cctccagcgg 840
cagcttcttg ccgggcaact tcccgcggtc agcatcgaga tccgtggtcg cgaagttgct 900
ggccacggcc acgatgttga agtcttcgtt gttctcggtg ggcttggcct cggccacagc 960
gatgcagatc agggcaaaca gaactttgac tcccatggtg gctggatccg agctcggtac 1020
caagcttcag ctgctcgagg ccggtctccc tatagtgagt cgtattaatt tcgataagcc 1080
aggttaacct gcattaatga atcggccaac gcgcggggag aggcggtttg cgtattgggc 1140
gctcttccgc ttcctcgctc actgactcgc tgcgctcggt cgttcggctg cggcgagcgg 1200
tatcagctca ctcaaaggcg gtaatacggt tatccacaga atcaggggat aacgcaggaa 1260
agaacatgtg agcaaaaggc cagcaaaagg ccaggaaccg taaaaaggcc gcgttgctgg 1320
cgtttttcca taggctccgc ccccctgacg agcatcacaa aaatcgacgc tcaagtcaga 1380
ggtggcgaaa cccgacagga ctataaagat accaggcgtt tccccctgga agctccctcg 1440
tgcgctctcc tgttccgacc ctgccgctta ccggatacct gtccgccttt ctcccttcgg 1500
gaagcgtggc gctttctcat agctcacgct gtaggtatct cagttcggtg taggtcgttc 1560
gctccaagct gggctgtgtg cacgaacccc ccgttcagcc cgaccgctgc gccttatccg 1620
gtaactatcg tcttgagtcc aacccggtaa gacacgactt atcgccactg gcagcagcca 1680
ctggtaacag gattagcaga gcgaggtatg taggcggtgc tacagagttc ttgaagtggt 1740
ggcctaacta cggctacact agaagaacag tatttggtat ctgcgctctg ctgaagccag 1800
ttaccttcgg aaaaagagtt ggtagctctt gatccggcaa acaaaccacc gctggtagcg 1860
gtggtttttt tgtttgcaag cagcagatta cgcgcagaaa aaaaggatct caagaagatc 1920
ctttgatctt ttctacgggg tctgacgctc agtggaacga aaactcacgt taagggattt 1980
tggtcatgag attatcaaaa aggatcttca cctagatcct tttaaattaa aaatgaagtt 2040
ttaaatcaat ctaaagtata tatgagtaaa cttggtctga cagttaccaa tgcttaatca 2100
gtgaggcacc tatctcagcg atctgtctat ttcgttcatc catagttgcc tgactccccg 2160
tcgtgtagat aactacgata cgggagggct taccatctgg ccccagtgct gcaatgatac 2220
cgcgagaccc acgctcaccg gctccagatt tatcagcaat aaaccagcca gccggaaggg 2280
ccgagcgcag aagtggtcct gcaactttat ccgcctccat ccagtctatt aattgttgcc 2340
gggaagctag agtaagtagt tcgccagtta atagtttgcg caacgttgtt gccattgcta 2400
caggcatcgt ggtgtcacgc tcgtcgtttg gtatggcttc attcagctcc ggttcccaac 2460
gatcaaggcg agttacatga tcccccatgt tgtgcaaaaa agcggttagc tccttcggtc 2520

```
ctccgatcgt tgtcagaagt aagttggccg cagtgttatc actcatggtt atggcagcac     2580

tgcataattc tcttactgtc atgccatccg taagatgctt ttctgtgact ggtgagtact     2640

caaccaagtc attctgagaa tagtgtatgc ggcgaccgag ttgctcttgc ccggcgtcaa     2700

tacgggataa taccgcgcca catagcagaa ctttaaaagt gctcatcatt ggaaaacgtt     2760

cttcggggcg aaaactctca aggatcttac cgctgttgag atccagttcg atgtaaccca     2820

ctcgtgcacc caactgatct tcagcatctt ttactttcac cagcgtttct gggtgagcaa     2880

aaacaggaag gcaaaatgcc gcaaaaaagg gaataagggc gacacggaaa tgttgaatac     2940

tcatactctt cctttttcaa tattattgaa gcatttatca gggttattgt ctcatgagcg     3000

gatacatatt tgaatgtatt tagaaaaata aacaaatagg ggttccgcgc acatttcccc     3060

gaaaagtgcc acctgacgtc taagaaacca ttattatcat gacattaacc tataaaaata     3120

ggcgtatcac gaggcccttt cgtctcgcgc gtttcggtga tgacggtgaa aacctctgac     3180

acatgcagct cccggagacg gtcacagctt gtctgtaagc ggatgccggg agcagacaag     3240

cccgtcaggg cgcgtcagcg ggtgttggcg ggtgtcgggg ctggcttaac tatgcggcat     3300

cagagcagat tgtactgaga gtgcaccata tggacatatt gtcgttagaa cgcggctaca     3360

attaatacat aaccttatgt atcatacaca tacgatttag gtgacactat a              3411
```

```
<210>  24
<211>  3309
<212>  DNA
<213>  Artificial Sequence

<220>
<223>  IGF-1 240A

<400>  24
gaaccagatc tcgtctcttt tttttttttt tttttttttt tttttttttt tttttttttt       60

tttttttttt tttttttttt tttttttttt tttttttttt tttttttttt tttttttttt      120

tttttttttt ttttttttca tctttttttt tttttttttt tttttttttt tttttttttt      180

tttttttttt tttttttttt tttttttttt tttttttttt tttttttttt tttttttttt      240

tttttttttt tttttttttt ttgaattcga gctcggtacc cttgaacaaa taacgtcgaa      300

tattaccaat gtttatttcg ttatcgtagt gtttaaagtg tttatttcgt tatcgtagtg      360

tttaaagtgt ttatttcgta aaaaaagtga cgtaagatca acaccaaaca ggtttgagta      420

gttacataga atagtacagc tcgatcgcca ctacaccgta aaagacgagg caccctccga      480

ggaggatgta agacatccag aacaaaggac gtgaaggaga tgaacacaag aagtttacat      540

gaaggaagac tcagaacccg tacagtcaca ccgcgacccg tgcctatctc gcccgacgaa      600

aacatccgaa gtcaccccgt gtcatgtaga ggtcagagga gtctagtgtc gaggccttcg      660
```

ttgtgagtag gtgttacgga cagactccac gggaggctta cgacctcggt atcggacacc 720
cgaacaactt cattttcggg gagccaggtg tgtgcttgac ttctcgtagg tggtcgagtc 780
ggggcgtttc ccagagacca ggtcgacacc acctcgacca cttccactcg ttcgtctcgc 840
ggtccatctt ctccacactt ctgctgtaca acacggagga gtcgacttgg ccactagagt 900
cgacatcctt tctcttcttc caatcatcag cttcgaggcc ggtctcccta tagtgagtcg 960
tattaatttc gataagccag gttaacctgc attaatgaat cggccaacgc gcggggagag 1020
gcggtttgcg tattgggcgc tcttccgctt cctcgctcac tgactcgctg cgctcggtcg 1080
ttcggctgcg gcgagcggta tcagctcact caaaggcggt aatacggtta tccacagaat 1140
caggggataa cgcaggaaag aacatgtgag caaaaggcca gcaaaaggcc aggaaccgta 1200
aaaaggccgc gttgctggcg tttttccata ggctccgccc ccctgacgag catcacaaaa 1260
atcgacgctc aagtcagagg tggcgaaacc cgacaggact ataaagatac caggcgtttc 1320
cccctggaag ctccctcgtg cgctctcctg ttccgaccct gccgcttacc ggatacctgt 1380
ccgcctttct cccttcggga agcgtggcgc tttctcatag ctcacgctgt aggtatctca 1440
gttcggtgta ggtcgttcgc tccaagctgg gctgtgtgca cgaacccccc gttcagcccg 1500
accgctgcgc cttatccggt aactatcgtc ttgagtccaa cccggtaaga cacgacttat 1560
cgccactggc agcagccact ggtaacagga ttagcagagc gaggtatgta ggcggtgcta 1620
cagagttctt gaagtggtgg cctaactacg gctacactag aagaacagta tttggtatct 1680
gcgctctgct gaagccagtt accttcggaa aaagagttgg tagctcttga tccggcaaac 1740
aaaccaccgc tggtagcggt ggtttttttg tttgcaagca gcagattacg cgcagaaaaa 1800
aaggatctca agaagatcct ttgatctttt ctacggggtc tgacgctcag tggaacgaaa 1860
actcacgtta agggattttg gtcatgagat tatcaaaaag gatcttcacc tagatccttt 1920
taaattaaaa atgaagtttt aaatcaatct aaagtatata tgagtaaact tggtctgaca 1980
gttaccaatg cttaatcagt gaggcaccta tctcagcgat ctgtctattt cgttcatcca 2040
tagttgcctg actccccgtc gtgtagataa ctacgatacg ggagggctta ccatctggcc 2100
ccagtgctgc aatgataccg cgagacccac gctcaccggc tccagattta tcagcaataa 2160
accagccagc cggaagggcc gagcgcagaa gtggtcctgc aactttatcc gcctccatcc 2220
agtctattaa ttgttgccgg gaagctagag taagtagttc gccagttaat agtttgcgca 2280
acgttgttgc cattgctaca ggcatcgtgg tgtcacgctc gtcgtttggt atggcttcat 2340
tcagctccgg ttcccaacga tcaaggcgag ttacatgatc ccccatgttg tgcaaaaaag 2400
cggttagctc cttcggtcct ccgatcgttg tcagaagtaa gttggccgca gtgttatcac 2460
tcatggttat ggcagcactg cataattctc ttactgtcat gccatccgta agatgctttt 2520
ctgtgactgg tgagtactca accaagtcat tctgagaata gtgtatgcgg cgaccgagtt 2580

gctcttgccc ggcgtcaata cgggataata ccgcgccaca tagcagaact ttaaaagtgc 2640

tcatcattgg aaaacgttct tcggggcgaa aactctcaag gatcttaccg ctgttgagat 2700

ccagttcgat gtaacccact cgtgcaccca actgatcttc agcatctttt actttcacca 2760

gcgtttctgg gtgagcaaaa acaggaaggc aaaatgccgc aaaaaaggga ataagggcga 2820

cacggaaatg ttgaatactc atactcttcc tttttcaata ttattgaagc atttatcagg 2880

gttattgtct catgagcgga tacatatttg aatgtattta gaaaaataaa caaatagggg 2940

ttccgcgcac atttccccga aaagtgccac ctgacgtcta agaaaccatt attatcatga 3000

cattaaccta taaaaatagg cgtatcacga ggccctttcg tctcgcgcgt ttcggtgatg 3060

acggtgaaaa cctctgacac atgcagctcc cggagacggt cacagcttgt ctgtaagcgg 3120

atgccgggag cagacaagcc cgtcagggcg cgtcagcggg tgttggcggg tgtcggggct 3180

ggcttaacta tgcggcatca gagcagattg tactgagagt gcaccatatg gacatattgt 3240

cgttagaacg cggctacaat taatacataa ccttatgtat catacacata cgatttaggt 3300

gacactata 3309

<210> 25
<211> 3184
<212> DNA
<213> Artificial Sequence

<220>
<223> IGF-1 120A

<400> 25
gaaccagatc tcgtctcttt tttttttttt tttttttttt tttttttttt tttttttttt 60

tttttttttt tttttttttt tttttttttt tttttttttt tttttttttt tttttttttt 120

tttttttttt tttttttgaa ttcgagctcg gtacccttga acaaataacg tcgaatatta 180

ccaatgttta tttcgttatc gtagtgttta aagtgtttat ttcgttatcg tagtgtttaa 240

agtgtttatt tcgtaaaaaa agtgacgtaa gatcaacacc aaacaggttt gagtagttac 300

atagaatagt acagctcgat cgccactaca ccgtaaaaga cgaggcaccc tccgaggagg 360

atgtaagaca tccagaacaa aggacgtgaa ggagatgaac acaagaagtt tacatgaagg 420

aagactcaga acccgtacag tcacaccgcg acccgtgcct atctcgcccg acgaaaacat 480

ccgaagtcac cccgtgtcat gtagaggtca gaggagtcta gtgtcgaggc cttcgttgtg 540

agtaggtgtt acggacagac tccacgggag gcttacgacc tcggtatcgg acacccgaac 600

aacttcattt tcggggagcc aggtgtgtgc ttgacttctc gtaggtggtc gagtcggggc 660

gtttcccaga gaccaggtcg acaccacctc gaccacttcc actcgttcgt ctcgcggtcc 720

atcttctcca cacttctgct gtacaacacg gaggagtcga cttggccact agagtcgaca 780

tcctttctct tcttccaatc atcagcttcg aggccggtct ccctatagtg agtcgtatta 840
atttcgataa gccaggttaa cctgcattaa tgaatcggcc aacgcgcggg gagaggcggt 900
ttgcgtattg ggcgctcttc cgcttcctcg ctcactgact cgctgcgctc ggtcgttcgg 960
ctgcggcgag cggtatcagc tcactcaaag gcggtaatac ggttatccac agaatcaggg 1020
gataacgcag gaaagaacat gtgagcaaaa ggccagcaaa aggccaggaa ccgtaaaaag 1080
gccgcgttgc tggcgttttt ccataggctc cgcccccctg acgagcatca caaaaatcga 1140
cgctcaagtc agaggtggcg aaacccgaca ggactataaa gataccaggc gtttccccct 1200
ggaagctccc tcgtgcgctc tcctgttccg accctgccgc ttaccggata cctgtccgcc 1260
tttctccctt cgggaagcgt ggcgctttct catagctcac gctgtaggta tctcagttcg 1320
gtgtaggtcg ttcgctccaa gctgggctgt gtgcacgaac cccccgttca gcccgaccgc 1380
tgcgccttat ccggtaacta tcgtcttgag tccaacccgg taagacacga cttatcgcca 1440
ctggcagcag ccactggtaa caggattagc agagcgaggt atgtaggcgg tgctacagag 1500
ttcttgaagt ggtggcctaa ctacggctac actagaagaa cagtatttgg tatctgcgct 1560
ctgctgaagc cagttacctt cggaaaaaga gttggtagct cttgatccgg caaacaaacc 1620
accgctggta gcggtggttt ttttgtttgc aagcagcaga ttacgcgcag aaaaaaagga 1680
tctcaagaag atcctttgat cttttctacg gggtctgacg ctcagtggaa cgaaaactca 1740
cgttaaggga ttttggtcat gagattatca aaaaggatct tcacctagat ccttttaaat 1800
taaaaatgaa gttttaaatc aatctaaagt atatatgagt aaacttggtc tgacagttac 1860
caatgcttaa tcagtgaggc acctatctca gcgatctgtc tatttcgttc atccatagtt 1920
gcctgactcc ccgtcgtgta gataactacg atacgggagg gcttaccatc tggccccagt 1980
gctgcaatga taccgcgaga cccacgctca ccggctccag atttatcagc aataaaccag 2040
ccagccggaa gggccgagcg cagaagtggt cctgcaactt tatccgcctc catccagtct 2100
attaattgtt gccgggaagc tagagtaagt agttcgccag ttaatagttt gcgcaacgtt 2160
gttgccattg ctacaggcat cgtggtgtca cgctcgtcgt ttggtatggc ttcattcagc 2220
tccggttccc aacgatcaag gcgagttaca tgatccccca tgttgtgcaa aaaagcggtt 2280
agctccttcg gtcctccgat cgttgtcaga agtaagttgg ccgcagtgtt atcactcatg 2340
gttatggcag cactgcataa ttctcttact gtcatgccat ccgtaagatg cttttctgtg 2400
actggtgagt actcaaccaa gtcattctga gaatagtgta tgcggcgacc gagttgctct 2460
tgcccggcgt caatacggga taataccgcg ccacatagca gaactttaaa agtgctcatc 2520
attggaaaac gttcttcggg gcgaaaactc tcaaggatct taccgctgtt gagatccagt 2580
tcgatgtaac ccactcgtgc acccaactga tcttcagcat cttttacttt caccagcgtt 2640
tctgggtgag caaaaacagg aaggcaaaat gccgcaaaaa agggaataag ggcgacacgg 2700

```
aaatgttgaa tactcatact cttccttttt caatattatt gaagcattta tcagggttat     2760
tgtctcatga gcggatacat atttgaatgt atttagaaaa ataaacaaat aggggttccg     2820
cgcacatttc cccgaaaagt gccacctgac gtctaagaaa ccattattat catgacatta     2880
acctataaaa ataggcgtat cacgaggccc tttcgtctcg cgcgtttcgg tgatgacggt     2940
gaaaacctct gacacatgca gctcccggag acggtcacag cttgtctgta agcggatgcc     3000
gggagcagac aagcccgtca gggcgcgtca gcgggtgttg gcgggtgtcg gggctggctt     3060
aactatgcgg catcagagca gattgtactg agagtgcacc atatggacat attgtcgtta     3120
gaacgcggct acaattaata cataacctta tgtatcatac acatacgatt taggtgacac     3180
tata                                                                  3184
```